# EUROPEAN PATENT SPECIFICATION

(11) **EP 1 570 839 B1**
(45) Date of publication and mention of the grant of the patent: **09.07.2008**
(21) Application number: 03810579.7
(22) Date of filing: 30.07.2003
(51) Int. Cl.: A61K 31/19, A61K 31/215, A61P 17/16, A61P 43/00, A61K 8/34, A61K 8/36, A61K 8/37, A61K 8/97, A61Q 1/02, A61Q 17/04, A61Q 19/02, A61Q 19/08, A61Q 19/10

(54) **COMPOSITION FOR PREPARATION FOR EXTERNAL USE ON SKIN AND METHOD OF USING THE SAME**
ZUSAMMENSETZUNGZUR HERSTELLUNG ZUR ÄUSSERLICHEN ANWENDUNG AUF DER HAUT SOWIE VERFAHREN ZU IHRER VERWENDUNG
COMPOSITION POUR UNE PREPARATION DESTINEE A UN USAGE EXTERNE SUR LA PEAU, ET METHODE D'UTILISATION S'Y RAPPORTANT

(30) Priority: 07.11.2002 JP 2002323502; 07.11.2002 JP 2002323503
(43) Date of publication of application: 07.09.2005
(73) Proprietor: Kosé Corporation, Chuo-ku, Tokyo 103-8251 (JP)
(72) Inventor: UEHARA, Shizuka, c/o KOSE CORPORATION, Tokyo 114-0005 (JP); HOSHINO, Taku, c/o KOSE CORPORATION, Tokyo 114-0005 (JP); MUTOU, Masakazu, c/o KOSE CORPORATION, Tokyo 114-0005 (JP); KAMEYAMA, Kumi, c/o KOSE CORPORATION, Tokyo 114-0005 (JP)
(74) Representative: Polypatent
(86) International application number: PCT/JP2003/009651
(87) International publication number: WO 2004/041236

(56) References cited:
- EP-A1- 0 970 693
- WO-A1-01/26670
- DE-A1- 19 821 971
- ES-A1- 2 107 976
- JP-A- 7 206 654
- JP-A- 7 238 059
- JP-A- 9 291 011
- JP-A- 11 060 467
- RU-C1- 2 026 683
- US-A- 4 668 433

## Description

### Technical Field

The present invention relates to an external preparation for skin, comprising a biologically-active ingredient containing a particular class of compound, and a particular class of medicinal ingredient, which is excellent in whitening effect and anti-aging effect.

### Background Art

Various medicinal ingredients have been used in conventional external preparation for skin such as milky lotaion, cream, lotion, facial mask, cleanser, dispersant, ointment, solution, aerosol, patch, adhesive skin patch and liniment for the purpose of giving predetermined medicinal benefits.

For example, whitening agents such as ascorbic acid, placenta extract, glutathione, hydroquinone have been used in order to prevent or improve skin tanning caused by sunburn and so forth, and skin pigmented spots or freckles caused by pigmentation. On the other hand, cell activators such as vitamin A, soybean extract and algae extract have been used in order to prevent or improve wrinkle and sagging of skin, and loosing tension or elasticity, caused by aging, UV exposure and so forth.

It has been also found that hot water extract or extracts in ethanol, hexane and so forth of stem, branch, leaf, etc. of *Cistaceae* such as *Cistus ladaniferus L. , Cistus creticus* L. , *Cistus monoperiensis* L., *Cistus salvifolius,* etc. have a strong suppressive action over melanin production, cell activation action and antibacterial action, and that these actions were ascribable to labdenic acids. It has been still also found that labd-7-en-15-oic acid, labd-8(17)-en-15-oic acid, and labd-B-en-15-oic acid, which are obtained by molecular distillation of the extracts or crude labdenic acids, have the strong suppressive action over melanin production, cell activation action, antibacterial action, etc. It has still also been found that salts, methyl and ethyl esters, and reduced products of these compounds have similar activities (Japanese Laid-Open Patent Publication "Tokkaihei" No. 11-302219, etc.).

LV-11552-B discloses a preparation (page 1, bottom table) suitable for skin application comprising:
- essence of labdone ("labdanuma esences")
- Cassis 345.

US-A-4 668 433 discloses two preparations (ex. 3E, 4A) suitable for skin application comprising:
- 8-alpha, 12-oxido-13,14,15,16 -tetra norlabdane ("Fixateur 404")
- Corps Cassis.

### DISCLOSURE OF THE INVENTION

An object of the present invention is to provide an external preparation for skin excellent in skin aesthetic effect, in particular whitening effect and/or anti-aging effect.

According to one aspect of the present invention, there is provided a composition comprising:
(A) one, or two or more compounds represented by formula (1) below: 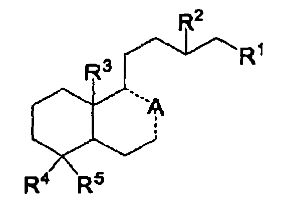 (in formula (1), R¹ represents -CH₂OH or COOR⁶, R⁶ represents hydrogen, a lower alkyl group having the number of carbon atoms of 1 to 3, or a cation capable of forming a salt with COO⁻, each of R² to R⁵ independently represents a hydrogen atom or methyl group, and ···A··· represents =C(CH₃)-, -C(CH₃)=, -C(=CH₂)-, -CH(CH₃)- or -C(OH) (CH₃)-); and
(B) one, or two or more medicinal ingredients selected from the group consisting of
*Glycyrrhiza glabra* extract, *Coix lachryma-jobi* extract, blackcurrant fruit extract, *Inula britannica* extract, cranberry fruit extract, *Mucuna birdwoodiana* extract, cactus extract, *Momordica grosvenorii* extract, astaxanthin and its derivatives,

The composition of the present invention is excellent as an external preparation for skin, in particular as a whitening external preparation for skin or an anti-aging external preparation for skin.

It is to be noted herein that the term "skin aesthetic" in this *specification* should be interpreted in a widest meaning typically including suppression of pigmentation; prevention and improvement of dullness of skin, tanning of skin due to sunburn; prevention and improvement of pigmented spots and freckles; and prevention and improvement of wrinkle, and should be understood that "whitening" and "anti-aging" are included within the scope of the term.

According to one embodiment of the present invention, there are provided composition, wherein the compound represented by formula (1) is a compound extracted from one, or two or more plants selected from the plant group consisting of *Cistaceae* including *Cistus ladaniferus L. , Cistus creticus* L., *Cistus monoperiensis* L. and *Cistus salvifolius,* or a compound prepared from the extracted compound; and a composition, being blended with one, or two or more extracts, comprising the compound represented by formula (1), selected from the plant group consisting of *Cistaceae* including *Cistus ladaniferus* L., *Cistus creticus* L., *Cistus monoperiensis* L. and *Cistus salvifolius.*

From other viewpoints of the present invention, there are provided the following non-therapeutic methods : a method of preventing melanin formation, comprising applying the above-described composition to skin; a method of whitening of skin, comprising applying the above described composition to skin; a method of anti-aging of skin, comprising applying the above-described composition to skin; a method of using the above-described composition as an external preparation for skin; a method of using one, or two or more selected from the medicinal ingredient group (B) enhancing medicinal benefit of one, or two or more of compounds represented by the above-described formula (1) ; a method of using one, or two or more selected from the medicinal ingredient group (B) enhancing melanin formation suppressive ability of one, or two or more of the compounds represented by the above-described formula (1); a method of using one, or two or more selected from the medicinal ingredient group (B) enhancing cell activation ability of one, or two or more of the compounds; and an enhancer of medicinal benefit of one, or two or more of compounds represented by the above-described formula (1), comprising one, or two or more selected from the medicinal ingredient group (B).

### MODES FOR CARRYING OUT THE INVENTION

Embodiments of an external preparation for skin utilizing the composition of the present invention will be described in details.

The external preparation for skin of the present invention comprises, as biologically-active ingredient, one, or two or more of compounds represented by formula (1) below (referred to as "ingredient (A)", hereinafter):

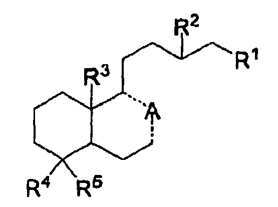

In the above-described formula (1), R¹ represents -CH₂OH or COOR⁶, R⁶ represents hydrogen, a lower alkyl group having the number of carbon atoms of 1 to 3, or a cation capable of forming a salt with COO⁻. The lower alkyl group having the number of carbon atoms of 1 to 3 may have a straight-chain form or may have a branched form. Examples of the alkyl group include methyl group, ethyl group, n-propyl group, and isopropyl group. Examples of the cation capable of forming a salt with -COO⁻ include Na⁺, K⁺ and NH₄⁺.

In the above-described formula (1), each of R² to R⁵ independently represents a hydrogen atom or methyl group, and ··· A ··· represents =C(CH₃)-, -C(CH₃)=, -C(=CH₂)-, -CH (CH₃) - or -C (OH) (CH₃) -).

The compounds represented by the above-described formula (1) can be extracted from plants. Extracts of plants are commercially available (e.g., Labdanum Absolute, product of Givaudan), and such commercial products can be used in the present invention. It is also allowable to obtain an acid having -COOH as R¹, convert it into methyl, ethyl and other esters, reduced product, or salt, and to use it as ingredient (A).

There is no limitation on species of plants from which the compounds represented by the above-described formula (1) are extracted, provided that they contain such compounds, and it is particularly advantageous to use plant body of *Cistus ladaniferus L*., *Cistus creticus* L., *Cistus monoperiensis* L., *Cistus salvifolius* (*Cistaceae)*. It is allowable to use them in a singular manner, or in combination of two or more species.

Portions of the plant body to be extracted are not specifically limited, and leaves, branches, barks, and so forth may be subjected to extraction. Extraction operation may be carried out immediately after collection of each portion, or may be carried out after each portion is collected and dried. The extraction is preferably carried out using one, or two or more solvents selected from water, lower alcohol, petroleum ether and hydrocarbon. The lower alcohol described herein means any alcohol having the number of carbon atoms of 1 to 4, and particularly preferable ones include methanol and ethanol. The petroleum ether may be any publicly-known ones, and any commercially-available ones may also be used. Examples of the hydrocarbon solvent include aliphatic hydrocarbon, alicyclic hydrocarbon and aromatic hydrocarbon, which exist in liquid form at normal temperature, wherein aliphatic hydrocarbon and aromatic hydrocarbon, which exist in liquid form at normal temperature, are particularly preferable, and among these, hydrocarbons such as hexane and toluene are preferable.

Although the extraction operation may differ depending on species of the plants and solvents to be used, it can generally be carried out by immersing a cut plant in the solvent at temperature ranging from room temperature to 50°C. It is also allowable to gently stir the solvent during the immersion. It is still also allowable to use an apparatus such as Soxhlet extractor. The extraction generally takes 3 hours to 48 hours or around. The extraction can also be carried out by crushing leaves, branches, trunks and so forth of the above-described plants, and by subjecting them to steam distillation or boiling in hot water. In these cases, the extract can be obtained by skimming gum state fraction floating on the water during the steam distillation or hot water extraction, and by separating the gum from impurities using a solvent used for the extraction. Thus-obtained extract contains 25 to 35% by mass (simply expressed as "%", hereinafter) of labdenic acids.

Although crude extract or commercial extract from the above-described plants may be used in their intact forms as ingredient (A), it is also allowable to further purify the crude extract or commercial extract to thereby obtain the compounds represented by the above-described formula (1), and use them as ingredient (A). A typical method of purifying the individual ingredients from the crude extract or commercial extract containing a mixture of labd-7-en-15-oic acid, labd-8(17)-en-15-oic acid and labd-8-en-15-oic acid will be described in details below, but the present invention is by no means limited to the method described below.

When the crude extract or commercial extract is subjected to molecular distillation under a reduced pressure of 0.1 to 0.5 mmHg, a fraction collected over a range from 160°C to 230°C may contain a mixture of labd-7-en-15-oic acid, labd-8(17)-en-15-oic acid and labd-8-en-15-oic acid. It is allowable to use the acid mixture without any modification as ingredient (A), or to prepare esters such as methyl ester, ethyl ester and so forth, salt, or reduced product as situation demands, and to use a mixture of these products.

It is still also allowable to separate three species of acids from the acid mixture. More specifically, the acid mixture is dissolved into ethanol, allowed to react in the presence of a catalytic amount of sulfuric acid to thereby convert it into an ester form, and subjected to silica gel chromatography using a silver nitrate-treated silica gel. A column is washed with hexane, and elution is carried out using an 1% ethyl acetate-hexane. Labd-8-en-15-oic acid ethyl ester is eluted first, which is followed by labd-7-en-15-oic acid ethyl ester and labd-8(17)-en-15-oic acid ethyl ester in this order. After the solvent is distilled off, pure preparations of the individual ethyl esters are obtained. Free acids can be obtained by hydrolyzing thus-obtained ethyl esters. It is further possible to obtain methyl esters by allowing the free acid to react with diazomethane.

Thus-obtained extracts of stems, branches, leaves and so forth of *Cistaceae* such as *Cistus ladaniferus* L., *Cistus creticus* L., *Cistus monoperiensis* L., *Cistus salvifolius* and so forth, and acid, methyl ester and ethyl ester obtained from the extracts, or mixtures containing two or more of them can be used as ingredient (A) in the present invention. That is, embodiments of the external preparations for skin of the present invention include those comprising, as ingredient (A), (i) hot water extract or organic solvent (ethanol, hexane, etc.) extract of *Cistaceae* such as *Cistus ladaniferus* L., *Cistus creticus* L., *Cistus monoperiensis* L., *Cistus salvifolius,* etc.; (ii) one, or two or more selected from labd-7-en-15-oic acid, labd-8(17)-en-15-oic acid, and labd-8-en-15-oic acid, obtained by molecular distillation of the extracts of above-described (i) or crude labdenic acids; (iii) salt, methyl ester or ethyl ester of the above-described (ii); and (iv) reduced products of the above-described (iii).

The amount of the compound represented by the above-described formula (1) in the external preparation for skin of the present invention is preferably 0.00001 to 5% as a dried solid content, and more preferably 0.0001 to 2%. Within these ranges, it is made possible to stably blend the plant extracts, and to exhibit an excellent skin brightening effect (in particular whitening and anti-aging effects). For the case where the extracted solution is used, concentration of the extracted solution is not limited at all, provided that the contents of the dried solid component, which is a solute, is adjusted within the above-described ranges.

In the present invention, ingredient (B) to be combined with ingredient (A) is one, or two or more selected from the agent listed below. It is to be noted that "derivatives" in the specific examples listed below include esters and salts:
*Glycyrrhiza glabra* extract, *Coix lachryma-jobi* extract, blackcurrant fruit extract, *Inula britannica* extract, *cranberry* fruit extract, *Mucuna birdwoodiana* extract, cactus extract, *Momordica grosvenorii* extract, astaxanthin and its derivatives,

*Glycyrrhiza glabra* extract, which can be used in the present invention, is obtained by extraction from root or rhizome (name of crude drug: *Kanzo)* of *Glycyrrhiza glabra* L. or congeneric plant, which belongs to Family Fabaceae, Glycyrrhiza, using an appropriate solvent. Oil-soluble extracts obtained by extracting *Glycyrrhiza glabra* of Chinese origin, Russian origin and so forth using a hydrophobic organic solvent are more preferable.

*Coix lachryma-jobi* extract, which can be used in the present invention, is obtained by extraction of uncoated seed (name of crude drug: *Yokuinin)* of Coix lacryma-jobi L.var. ma-yuen Stapf which belongs to Family Gramineae, *Coix* L., using an appropriate solvent.

Blackcurrant fruit extract, which can be used in the present invention, is a fruit extract obtained by extraction of fruit of blackcurrant (Japanese name: kurofusasuguri, French name: cassis, *Ribes nigrum* L.) which belongs to Family Ribesoideae, Ribes L., using an appropriate solvent, or a squeezed fruit juice.

*Inula britannica* extract, which can be used in the present invention, is obtained by extraction of flower (name of crude drug: *Senpukuka)* of *Inula Britannica* L. which belongs to Family Asteraceae, *Inula* L., using an appropriate solvent.

Cranberry fruit extract, which can be used in the present invention, is a fruit extract obtained by extraction of fruit of cranberry (Japanese name: turukokemomo, *Vaccinium macrocorpon* aiton or *vaccinium oxycoccus*) which belongs to Ericaceae Vaccinium, using an appropriate solvent, or a squeezed fruit juice.

*Mucuna birdwoodiana* extract, which can be used in the present invention, is obtained by extraction from stem portion (name of crude drug: *Kkeikettou*) of *Spatholobus suberectus, Mucuna* birdwoodiana, *Mucuna Birdwoodiana* Tutcher, *Milletia dielsiana* Harms (Chinese name: *Koukagantoutou)* or *Milletia nitida* Benth (*Ryouyougantoutou*)*,* which are plants belongs to Family Fabaceae, using an appropriate solvent.

Cactus extract, which can be used in the present invention, may be those obtained from plants of Family Cactaceae, and obtained typically from stem, root, fruit and so forth of *Opuntia ficus-indica* (L.)Mill, *Opuntia dillenii* Haw, *Opuntia Streptacantha* and so forth, using an appropriate solvent.

*Momordica grosvenorii* extract, which can be used in the present invention, is obtained by extracting dried fruit (Chinese name: *Rakanka)* of *Momordica grosvenorii* Swingle which belongs to Family Cucurbitaceae, *Mormodica,* using an appropriate solvent.

Astaxanthin and its derivatives, which cqan be used in the present invention, can be obtained by extracting natural materials such as *Euphausia,* salmon, trout, *Adonis,* red yeast and so forth, using an appropriate solvent, or by chemical synthesis. For example, it is allowable to use an extract of astaxanthin obtained by adding an extraction solvent to *Euphausia similis* G.O. or the like, extracting it, and filtering the solution, or to use purified astaxanthin obtained by further removing the extraction solvent from the extract, allowing chemical reactions such as hydrogen-addition or hydrolysis to proceed if situation demands, and deodorizing and purifying the product by means of molecular distillation, column chromatography or high-performance liquid chromatography. Astaxanthin is a kind of carotenoid represented by the formula below, and derivatives thereof are exemplified by monoester selected from fatty acid esters of astaxanthin, and same kind or different kinds of diesters.

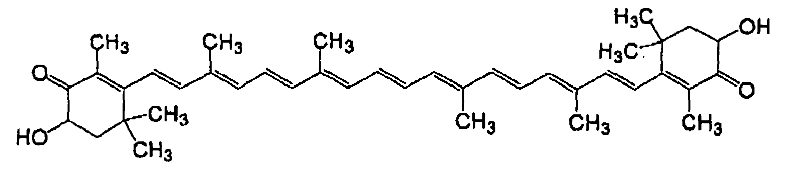

Example of the extract solvent to be used for the preparation of the ingredients which belong to the above-described group (B) include water, lower monohydric alcohols (methanol, ethanol, 1-propanol, 2-propanol, 1-butanol, 2-butanol, etc.), liquid polyhydric alcohols (glycerin, etc.), hydrocarbons (benzene, hexane, pentane, etc.), ketones (acetone, methyl ethyl ketone, etc.), ethers (diethyl ether, tetrahydrofuran, dipropyl ether, etc.) and acetonitrile, from which one, or two or more species can be used. An example of preferable method of extraction is such that extraction is carried out at room temperature or under heating for 1 to 5 days, using ethanol or 1, 3-butylene glycol having a water content of 0 to 100 vol% , the mixture is filtered, and the obtained filtrate is allowed to stand further for a week or around for aging, and is filtered again.

Of the above-described ingredient (B), *Glycyrrhiza glabra* extract, *Coix lachryma-jobi* extract, blackcurrant fruit extract, *Inula britannica* extract, cranberry fruit extract, *Mucuna birdwoodiana* extract, cactus extract, *Momordica grosvenorii* extract and astaxanthin and its derivatives (all of which will be referred to as ingredient (C2)) can exhibit medicinal benefits mainly as a whitening agent even when they are used independently, but, used in combination with the above-described ingredient (A), may exhibit excellent whitening and/or anti-aging effects which cannot be obtained by the independent use.

Of the above-described ingredient (B2), *Glycyrrhiza glabra* extract, *Mucuna birdwoodiana* extract, *Momordica grosvenorii* extract, astaxanthin and its derivatives (all of which will be referred to as ingredient (D2)) can exhibit medicinal benefits mainly as an antioxidant even when they are used independently, but, used in combination with the above-described ingredient (A), may exhibit excellent whitening and/or anti-aging effects which cannot be obtained by the independent use.

Of the above-described ingredient (B2), *Glycyrrhiza glabra* extract and *Mucuna birdwoodiana* extract (both of which will be referred to as ingredient (E2)) can exhibit medicinal benefits mainly as antiinflammatory agent even when they are used independently, but, used in combination with the above-described ingredient (A), may exhibit excellent whitening and/or anti-aging effects which cannot be obtained by the independent use.

Of the above-described ingredient (B2), cactus extract, *Momordica grosvenorii* extract, astaxanthin and its derivatives (all of which will be referred to as ingredient (F2)) can exhibit medicinal benefits mainly as a cell activator even when they are used independently, but, used in combination with the above-described ingredient (A), may exhibit excellent whitening and/or anti-aging effects which cannot be obtained by the independent use.

Of the above-described ingredient (B2), *Glycyrrhiza glabra* extract, *Coix lachryma-jobi* extract, blackcurrant fruit extract, *Inula britannica* extract, cranberry fruit extract, *Mucuna birdwoodiana* extract, cactus extract and *Momordica grosvenorii* extract (all of which will be referred to as ingredient (G2)) can exhibit medicinal benefits mainly as a moisturizer even when they are used independently, but, used in combination with the above-described ingredient (A), may exhibit excellent whitening and/or anti-aging effects which cannot be obtained by the independent use.

Preferable blending amount of the above-described ingredient (B) in the external preparation for skin of this embodiment, possibly differ by species (ingredient (C) to ingredient (G)), are preferably adjusted within ranges shown below. Within these ranges, combination with ingredient (A) makes it possible to exhibit larger skin brightening effects (in particular, whitening and/or anti-aging effects) without adversely affecting long-term stability of formulation and ingredient (A) in the formulation.

Of ingredient (B2), those exhibiting a plurality of medicinal benefits were repetitively exemplified also as ingredients (C2) to (G2), and it is to be noted that, for those repetitively exemplified, the widest ranges, out of the individual preferable ranges of amount of addition, will be understood as the most preferable ranges of blending amount.

Blending amount of the above-described ingredient (C2) in the external preparation for skin of this embodiment is preferably 0.00001 to 10%, and more preferably 0. 0001 to 5%. When the extract is used in a form of extracted solution, it is all enough to adjust the dried solid content within these ranges. Adjustment within these ranges is successful in obtaining an external preparation for skin exhibiting a more excellent whitening and/or anti-aging effects and giving a good feel in use.

Blending amount of the above-described ingredient (D2) in the external preparation for skin of this embodiment is preferably 0.00001 to 5%, and more preferably 0.0001 to 3%. When the extract is used in a form of extracted solution, it is all enough to adjust the dried solid content within these ranges. Adjustment within these ranges is successful in obtaining an external preparation for skin exhibiting an excellent antioxidative effect, a more excellent whitening and/or anti-aging effects, and giving a good feel in use.

Blending.amount of the above-described ingredient (E2) in the external preparation for skin of this embodiment is preferably 0.00001 to 5%, and more preferably 0.0001 to 3%. When the plant extract is used in a form of extracted solution, it is all enough to adjust the dried solid content within these ranges. Adjustment within these ranges is successful in obtaining an external preparation for skin exhibiting an antiinflammatory effect, an excellent whitening and/or anti-aging effects, and giving a good feel in use.

Blending amount of the above-described ingredient (F2) in the external preparation for skin of this embodiment is preferably 0. 00001 to 5%, and more preferably 0. 0001 to 3%. When the extract is used in a form of extracted solution, it is all enough to adjust the dried solid content within these ranges. Adjustment within these ranges is successful in obtaining an external preparation for skin exhibiting a more excellent improving effect for roughened skin, exhibiting an excellent whitening and/or anti-aging effect, and giving a good feel of use.

Blending amount of blending of the above-described ingredient (G2) in the external preparation for skin of this embodiment is preferably 0.001 to 25%, and more preferably 0.01 to 20%. Adjustment within these ranges is successful in obtaining an external preparation for skin exhibiting a more excellent moisturizing effect, an excellent whitening and/or anti-aging effect, and giving a good feel in use.

The external preparation for skin of the present invention can be prepared according to general procedures, by blending the above-described ingredient (A) and ingredient (B), which are essential ingredients, to various forms of base materials known as general external preparation for skin.

Form of blending of the external preparation for skin is not specifically limited, and any cosmetic formulation and external medicine having forms of milk lotion, cream, lotion, face mask, cleanser, makeup cosmetic formulation, dispersion liquid, ointment, solution, aerosol, patch, adhesive skin patch and liniment are allowable.

Beside the above-described essential ingredients, the external preparation for skin of the present invention may also be added with various ingredient generally used for cosmetics, for medicated cosmetics, external preparation for skin and so forth, such as water, alcohol, oil-base material, surfactant, viscous agent, powder, cheleting agent, pH adjustor, various medicinal ingredients, extracts of animal, plant and bacterial origins, perfume, within amount of ranges without impairing the effects of the present invention. Among these, specific examples will be shown below.

As for alcohol, monohydric ones such as ethanol, and polyhydric ones such as glycerin and 1,3-butylerie glycol can be used within a range not overlapping the essential ingredients, for purposes of solubilization, refreshing, preserving, moisturizing and so forth.

Oil-base material can be used irrespective of its origin and properties, for the purpose of improving handerability and feel of use. Specific examples include liquid paraffin, squalane, triglyceride oil, ester oil, waxes, fatty acids, higher alcohol, silicone oil, fluorine-containing oil and various waxes.

Surfactant is used for emulsifying or solubilizing the oil-base material and so forth, and for which anionic, cationic, nonionic and amphoteric surfactants are available.

As viscous agent, it is possible to use carboxyvinyl polymer, carragenan, agar, xanthane gum, dextrin fatty acid ester, organo-modified clay mineral and so forth, irrespective of that they are chemically-synthesized ones or derived from natural products. These ingredients can be used not only for adjusting viscosity of the system, but also for gellation, moisturizing and film formation.

Powder is not limited by its geometry, grain size, presence or absence of porosity, and crystal structure, and even may be made into composite material or surface-treated for the purpose of improving handerability and feel of use. Inorganic powders such as talc, mica, sericite and silisic anhydride, etc., organic powders such as nylon powder, etc., pearl pigments such as fish scale foil and bismuth oxychloride, inorganic pigments such as iron oxide, carbon black, ultramarine blue, etc., tar colorant and its lake, natural colorant, titanium oxide, fine titanium oxide powder, zinc oxide, fine zinc oxide powder can be used depending on purpose of use. In particular, use of fine titanium oxide powder and fine zinc oxide powder is preferable in view of further improving the effect of the present invention.

In order to prevent quality of ingredients in the system from degrading, it is also allowable to use cheleting agent such as EDTA, or pH adjustor based on buffer such as lactic acid-sodium lactate.

As medicinal ingredient, various products containing vitamin, hormone, and extracts of animal, plant, and bacterial origins are exemplified. For example, antibacterial agent can be used for preventing or improving acme and so forth, and examples of which include benzoic acid, sodium benzoate, paraoxy benzoate ester, parachloro metacresol, benzalkonium chloride, phenoxyethanol and isopropyl methyl phenol. By blending of these ingredients, it is made possible to suppress pigmentation caused by bacterial skin inflammation such as acme, and to exhibit still higher whitening and/or skin brightening effects, and anti-aging effect.

Active oxygen removing agent is used typically for suppressing generation of UV-induced lipid peroxide, and typically exemplified by vitamin B which include superoxide dismutase, catechin and its derivatives, thiamins (thiamin hydrochlorate, thiamin sulfate), riboflavins (riboflavin, riboflavin acetate, etc.), pyridoxines (pyridoxine hydrochlorate, pyridoxine dioctanoate, etc.), nicotinic acids (nicotinic amide, benzyl nicotinate, etc.) and so forth. By blending these active oxygen removing agent, it is made possible to suppress darkening of skin, and to exhibit still higher whitening and/or skin brightening effects, and anti-aging effect.

Examples of other ingredients for combined use, possibly improving the effects of the present invention, include protein or derivatives or hydrolyzates and salts thereof (collagen, elastin, keratin, etc.), mucopolysaccharide and its derivatives (hyaluronic acid, chondroitin sulfate, etc.), amino acids and their derivatives (hystidine, serine, glycine, teanin, aspartic acid, arginine, pyrrolidone carboxylic acid, etc.), sugars (sorbitol, erythritol, trehalose, inositol, glucose, xylitol, sucrose and its derivatives, dextrin and its derivatives, honey, etc.), D-pantenol and its derivatives, glycolipid, ceramid, *Angelica* extract, avocado extract, hot spring water, *Marrow* extract, *White nettle* extract, ononis extract, oats extract, *Gardenia* extract, *Sasa Albo-marginata* extract, Burdock root extract, wheat extract, *Saponaria* extract, Filipendula extract, ginger extract, *Mentha piperita* (peppermint) extract, *Thymus vulgaris* L. (thyme) extract, camellia extract, *Tormentilla* extract, parsley extract, mint extract, hamamelis extract, rose extract, *hinoki* cypress extract, sunflower extract, butcher's bloom extract, prune extract, *Sponge gourd* extract, *Linden* extract, pine extract, Quince seed extract, mutin, Cornflower extract, lavender extract, apple extract, and *Gentiana* extract.

It may be also made possible to give still higher whitening and/or skin brightening effects, and to make skin clearer, by blending agents capable of sealing the surface of skin, such as jojoba oil, macadamia nut oil, olive oil, Persic oil, persic oil, safflower oil, sunflower oil, avocado oil, meadow foam oil, camellia oil, almond oil, perilla oil, sesame, *Borago officinalis* (borage) oil, cacao butter and shea butter (synonym, name of crude drug, etc. were given in the parentheses).

Circulation accelerator is used for accelerating blood circulation of skin so as to promote discharge of melanin, which can be exemplified by capsicum tincture, γ-oryzanol and so forth, and enzymes such as lipase and papain. Blending of these ingredients is successful in exhibiting still higher whitening and/or skin brightening effects.

### Examples

Next, reference examples, test examples and examples will be described hereinafter, however, the present invention is by no means limited to the examples.

### [Example 1] Preparation of Cistus ladaniferus L. Extract

Twenty kilograms of crushed leaves and twigs of *Cistus ladaniferus L.,* a plant of *Cistaceae,* were deoiled by steam distillation. The mixture was extracted with 200 kg of n-hexane, the obtained extract was distilled under reduced pressure so as to remove low-boiling-point components, to thereby obtain 150 g of extract in a solid to paste form.

### [Example 2] Preparation of labdenic acids, and their methyl esters and ethyl esters

Ten grams of commercial Labdanum Absolute (product of Givaudan) were subjected to molecular distillation under reduced pressure (0.1 mmHg), and a fraction (4.3 g) was collected over a range from 180°C to 220°C. The fraction was found to contain compound 1 (labd-8-en-15-oic acid), compound 4 (labd-7-en-15-oic acid) and compound 7 (labd-8 (17)-en-15-oic acid) (the mixture is referred to as acid mixture, hereinafter), shown below. One gram of the acid mixture was dissolved into 2 ml of ether, an ether solution of diazomethane is dropped therein, and thereby 0.96 g of a methyl ester product was obtained (the methyl ester product is referred to as methyl ester mixture, hereinafter). Similarly, 10 g of the acid mixture was dissolved into 100 mL of ethanol, allowed to proceed esterification under the presence of a sulfuric acid catalyst, to thereby obtain 9.5 g of ethyl ester product (the ethyl ester product is referred to as ethyl ester mixture, hereinafter).

Next, the ethyl ester mixture was chromatographed on silica gel to thereby separate it into three acids. More specifically, 10 g of the ethyl ester mixture was dissolved into 100 mL of hexane, injected to a silver-nitrate-treated silica gel column, which was followed by injection of solvents and elution. The solvents injected were hexane for the beginning, and hexane added with 1 vol% of ethyl acetate for the next. Compound 3 (labd-8-en-15-oic acid ethyl ester) was eluted first, and compound 6 (labd-7-en-15-oic acid ethyl ester) and compound 9 (labd-8(17)-en-15-oic acid ethyl ester) followed in this order. The solvent were removed from each of the eluates, to thereby obtain pure products of the individual ethyl esters (0.83 g, 0.16 g and 0.63 g in this order of elution). Thus-obtained ethyl ester products were hydrolyzed according to a general method, to thereby obtain free acids. The free acids were further added by dropping with an ether solution of diazomethane, and the solvent was distilled off, to thereby obtain the methyl ester products.

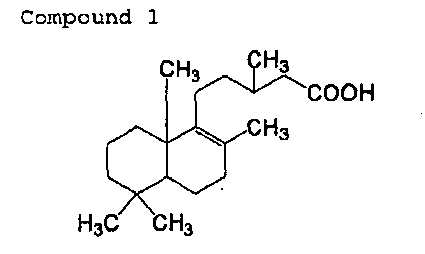

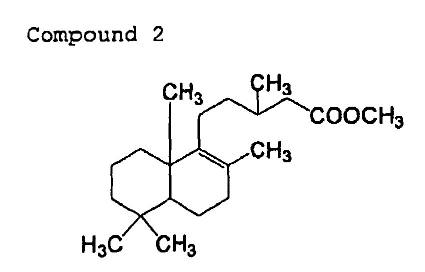

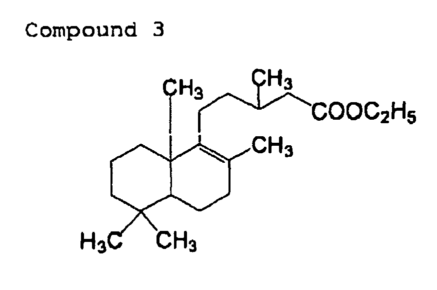

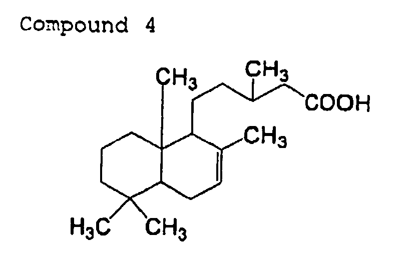

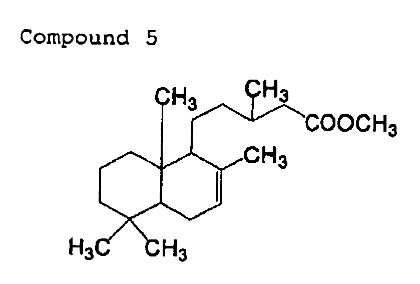

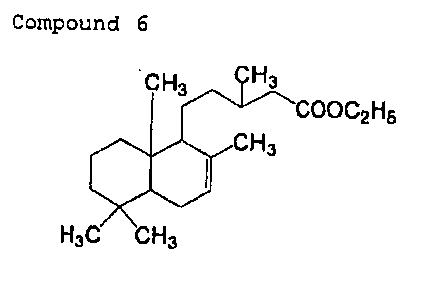

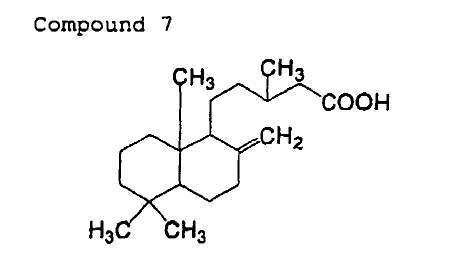

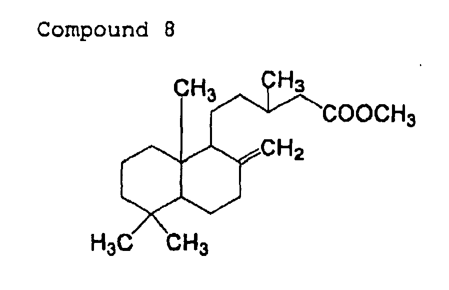

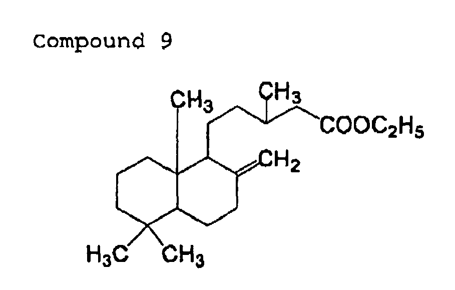

Then 4.3 g each of the ethyl ester products obtained in Example 2 was dissolved into 10 mL of ethanol, added with 0.2 g of a 5%-palladium carbon catalyst so as to proceed hydrogen addition reaction, to thereby obtain 4.1 g of compound 11. The product was further hydrolyzed to obtain compound 10.

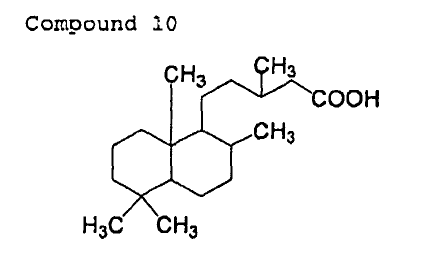

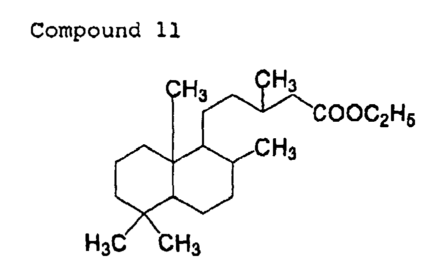

### [Example 3] Melanin Generation Suppression and Cell Survival Rate Test based on Cell Culture

Murine cultured 816 melanoma cells were used. An appropriate quantity of a 10% FBS-containing MEM medium was placed in two 6-well plates, the B16 melanoma cells were seeded therein and allowed to stand at 37°C under a carbon dioxide concentration of 5 vol%. Next day, a sample preparation solution was added and mixed therewith so as to adjust the final concentrations of the *Cistus ladaniferus* L. extract obtained in Example 1, and labdenic acids and methyl esters and ethyl esters thereof obtained in Example 2 to 0 (reference), 5 and 10 µg/mL, and of lily extract (product of Maruzen Pharmaceuticals Co., Ltd.), which is a known whitening agent, to 0 and 100 µg/mL. The medium was exchanged on the fifth day of culture, and the sample preparation solution was added again. The medium was removed next day, the cells were collected from one plate after washing them using a phosphate buffer (pH7), and degree of whitening of the cultured B16 melanoma cells was evaluated according to the criteria shown below.

Similar test was conducted, as a comparative example, also using *Coix lachryma-jobi* extract (100 µg/mL), already known to have a suppressive effect over melanin formation.

*Coixlachryma-jobi* extract was obtained by adding 100 mL of a 70 vol% water-containing ethanol to 10 g of *Coixlachryma-jobi* (Japan Pharmacopoeia), carrying out extraction at room temperature for 3 days, and by filtering the mixture. Dry solid content of the *Coixlachryma-jobi* extract was found to be 0.8%.

### (Criteria for Judgment)

++: distinctively stronger whiteness over the reference;
+: apparently stronger whiteness over the reference;
±: slightly stronger whiteness over the reference; and
-: remained unchanged.

On the other plate, the cells were fixed with formalin, and dyed by adding an 1% crystal violet solution. Cell survival rates for the individual sample concentrations were measured using a monocellator (product of Olympus Corporation). Results are shown in Table 1-1.

**Table 1-1 (Results)**

| | Final concentration (µg/mL) | Additional concentration of lily extract (µg/mL) | Degree of whiteness | Cell survival rate (%) |
|---|---|---|---|---|
| Cistus ladaniferus L. Extract*1 | 10 | 100 | ++ | 105 |
| Acid mixture*2 | 5 | 100 | ++ | 120 |
| Methyl ester mixture*2 | 5 | 100 | ++ | 98 |
| Ethyl ester mixture*2 | 5 | 100 | ++ | 89 |
| Compound 1 | 5 | 100 | ++ | 92 |
| Compound 4 | 5 | 100 | ++ | 94 |
| Compound 7 | 5 | 100 | ++ | 95 |
| Coix lachrymal-jobi extract*3 | 100 | 100 | ++ | 94 |
| Cistus ladaniferus L. Extract*1 | 20 | 0 | + | 100 |
| Acid mixture *2 | 10 | 0 | + | 92 |
| Methyl ester mixture*2 | 10 | 0 | + | 90 |
| Ethyl ester mixture*2 | 10 | 0 | + | 90 |
| Compound 1*2 | 10 | 0 | + | 94 |
| Compound 4*2 | 10 | 0 | + | 93 |
| Compound 7*2 | 10 | 0 | + | 98 |
| Coix lachrymal-jobi extract*3 | 200 | 0 | ± | 100 |
| Lily extract | 0 | 200 | ± | 100 |

| | | | | |
|---|---|---|---|---|
| *1 prepared in Example 1 *2 prepared in Example 2 *3 prepared in Example 3 *4 manufactured by Maruzen Pharmaceuticals Co., Ltd. | | | | |

As is understandable from the results shown in Table 1-1, *Cistus ladaniferus* L. extract and labdenic acids and methyl esters and ethyl esters thereof were found to have suppressive effects over melanin formation even when used in a singular manner, and to have only a small toxicity over the cultured B16 melanoma cell. It was also found that combined use with the lily extract, which is a known whitening agent, resulted in a synergistic whitening effect, as compared with the case of singular use. It is therefore expected that application of formulations of *Cistus ladaniferus* L. extract and labdenic acids and methyl esters and ethyl esters thereof, combined with lily extract, exhibits an extremely excellent suppressive effect over melanin formation, effectively suppresses sunburn-induced darkening of skin, pigmented spots, freckles and so forth, and gives whitening and skin brightening effects.

### [Example 4: Cream] (out of scope of protection)

The individual creams having the formulations shown in Table 2-1 were prepared.

First, ingredients (1) to (6) and (12) were mixed and kept at 70°C, and added with ingredient (16) again kept at 70°C by heating. The mixture was further added and mixed with ingredients (7) to (11) and (13) to (15), and then cooled to thereby obtain the creams.

Whitening and skin brightening effects of thus-obtained creams were investigated according to the test method shown below.

### (Test Method)

A panel of 15 female subjects aged from 27 to 54 were employed for each of the sample creams, and made them apply a proper quantity of the creams on their faces after washing twice a day, in the morning and at night, over 12 weeks, and effects of whitening and skin brightening by the application were evaluated based on the criteria below. Results of the evaluation were represented by the number of subjects in the panel relevant to each evaluation.

**(Criteria for Evaluation)**

| <Evaluation> | <Description> |
|---|---|
| Effective | wrinkle of skin became non-distinctive |
| Little effective | wrinkle of skin became a little non-distinctive |
| Non-effective | remained unchanged |

**Table 2-1 (Formulations and Results)**

| | Ingredient | Example | | | | | |
|---|---|---|---|---|---|---|---|
| | | 1 | 2 | 3 | 4 | 5 | 6 |
| (1) | bleached bees wax | 6.0 | 6.0 | 6.0 | 6.0 | 6.0 | 6.0 |
| (2) | cetanol | 5.0 | 5.0 | 5.0 | 5.0 | 5.0 | 5.0 |
| (3) | reduced lanolin | 5.0 | 5.0 | 5.0 | 5.0 | 5.0 | 5.0 |
| (4) | squalane | 30.0 | 30.0 | 30.0 | 30.0 | 30.0 | 30.0 |
| (5) | lipophilic glyceryl monostearate | 4 .0 | 4.0 | 4.0 | 4.0 | 4.0 | 4.0 |
| (6) | polyoxyethylen (20) sorbitan monolaurate | 2.0 | 2.0 | 2.0 | 2.0 | 2.0 | 2.0 |
| (7) | *Cistus ladaniferus L.* extract*1 | 0.1 | 0.1 | 0.1 | 0.1 | 0.1 | 0.1 |
| (8) | *Morus bombycis* extract*2 | 1.0 | - | - | - | - | - |
| (9) | natural Vitamin E | - | 0.5 | - | - | - | - |
| (10) | dipotassium glycyrrhiziate*4 | - | - | 0.1 | - | - | - |
| (11) | yeast extract*5 | - | - | - | 0.5 | - | - |
| (12) | 2-ethylhexyl paramethoxycinnamate*6 | - | - | - | - | 2.5 | - |
| (13) | hydrogen-added soybean phospholipid*7 | - | - | - | - | - | 0.5 |
| (14) | antiseptic | q.s. | q.s. | q.s. | q.s. | q.s. | q.s. |
| (15) | perfume | q.s. | q.s. | q.s. | q.s. | q.s. | q.s. |
| (16) | pure water | balance | balance | balance | Balance | balance | balance |
| *8 | effective | 13 | 13 | 14 | 12 | 12 | 13 |
| | little-effective | 2 | 1 | 1 | 3 | 2 | 2 |
| | non-effective | 0 | 1 | 0 | 0 | 1 | 0 |

| | | | | | | | |
|---|---|---|---|---|---|---|---|
| *1 prepared in Example 1 *2 product of Maruzen Pharmaceuticals Co., Ltd. *3 product of Eisai Co., Ltd. *4 product of Maruzen Pharmaceuticals Co., Ltd. *5 product of Maruzen Pharmaceuticals Co., Ltd. *6 product of BASF *7 Nikko Chemicals Co. Ltd. *8 Whitening and skin brightening effect | | | | | | | |

**Table 2-1 (Formulations and Results)**

| | Ingredient | Comparative Example | | | | | | | |
|---|---|---|---|---|---|---|---|---|---|
| | | 1 | 2 | 3 | 4 | 5 | 6 | 7 | 8 |
| (1) | bleached bees wax | 6.0 | 6.0 | 6.0 | 6.0 | 6.0 | 6.0 | 6.0 | 6.0 |
| (2) | cetanol | 5.0 | 5.0 | 5.0 | 5.0 | 5.0 | 5.0 | 5.0 | 5.0 |
| (3) | reduced lanolin | 5.0 | 5.0 | 5.0 | 5.0 | 5.0 | 5.0 | 5.0 | 5.0 |
| (4) | squalane | 30.0 | 30.0 | 30.0 | 30.0 | 30.0 | 30.0 | 30.0 | 30.0 |
| (5) | lipophilic glyceryl monostearate | 4.0 | 4.0 | 4.0 | 4.0 | 4.0 | 4.0 | 4.0 | 4.0 |
| (6) | polyoxyethylen (20) sorbitan monolaurate | 2.0 | 2.0 | 2.0 | 2.0 | 2.0 | 2.0 | 2.0 | 2.0 |
| (7) | *Cistus ladaniferus L. extract*1* | 0.2 | - | - | - | - | - | - | - |
| (8) | *Morus bombycis* extract*2 | - | 2.0 | - | - | - | - | - | - |
| (9) | natural vitamin E | - | - | 1.0 | - | - | - | - | - |
| (10) | dipotassium glycyrthiziate*4 | - | - | - | 0.2 | - | - | - | - |
| (11) | yeast extract*5 | - | - | - | - | 1.0 | - | - | - |
| (12) | 2-ethylhexyl paramethoxycinnamate *6 | - | - | - | - | - | 5.0 | - | - |
| (13) | hydrogen-added soybean phospholipid*7 | - | - | - | - | - | - | 1.0 | - |
| (14) | antiseptic | q.s. | q.s. | q.s. | q.s. | q.s. | q.s. | q.s. | q.s. |
| (15) | perfume | q.s. | q.s. | q.s. | q.s. | q.s. | q.s. | q.s. | q.s. |
| (16) | pure water | balance | balance | balance | Balance | balance | balance | balance | balance |
| *8 | effective | 7 | 4 | 2 | 4 | 4 | 2 | 5 | 0 |
| | little-effective | 8 | 8 | 8 | 8 | 7 | 6 | 8 | 5 |
| | non-effective | 0 | 3 | 5 | 3 | 4 | 7 | 2 | 10 |

| | | | | | | | | | |
|---|---|---|---|---|---|---|---|---|---|
| *1 prepared in Example 1 *2 product of Maruzen Pharmaceuticals Co., Ltd. *3 product of Eisai Co., Ltd. *4 product of Maruzen Pharmaceuticals Co., Ltd. *5 product of Maruzen Pharmaceuticals Co., Ltd. *6 product of BASF *7 Nikko Chemicals Co. Ltd. *8 Whitening and skin brightening effect | | | | | | | | | |

It was made clear from the results shown in Table 2-1 that, when applied to the skin, the creams of the present invention in which *Cistus ladaniferus* L. extract and various ingredients as ingredient (B) (ingredients (8) to (13)) were used together were more successful in preventing and improving "darkening" of skin, and in making the skin more beautiful as compared with the comparative products.

### [Example 5: Cell Activation Test based on Cell Culture] (out of protection scope)

Human neonatal fibroblast NB1RGB was used. An appropriate quantity of medium was placed in a 24-well plate, the fibroblast NB1RGB was seeded therein and allowed to stand at 37°C under a carbon dioxide concentration of 5 vol%. Next day, a sample preparation solution was added and mixed therewith so as to adjust the final concentrations of the *Cistus ladaniferus* L. extract obtained in Example 1, and labdenic acids and methyl esters and ethyl esters thereof obtained in Example 2 to 0 (reference), 5 and 10 µg/mL, and of *Panax ginseng* extract (product of Maruzen Pharmaceuticals Co., Ltd.), which is a known cell activator, to 0 and 100 µg/mL. The medium was exchanged on the fourth day of culture, and the sample preparation solution was added again. The medium was removed next day, the cells were washed using a phosphate buffer and collected, and evaluated in terms of cell proliferation rate based on comparison of the number of fibroblast NB1RGB cells grown in the individual sample preparation solutions with that of the reference.

Similar test was conducted, as a comparative example, also using soybean extract (100 µg/mL), already known to have a cell activation effect. Soybean extract was obtained by adding 100 mL of a 70 vol% water-containing ethanol to 10 g of soybean seed, carrying out extraction at room temperature for 3 days, and by filtering the mixture. Dry solid content of the soybean extract was found to be 0.5%.

### (Criteria for Evaluation)

The number of cells grown in each sample preparation solution was compared with the number of cells of the reference, and cell activation effect was evaluated using cell proliferation ratio as an index. The number of cells was counted using a blood cell counter plate.

**Table 3-1 (Results)**

| | Final concentration (µg/mL) | Additional concentration of *Panax ginseng* extract (µg/mL) | Cell-activation rate (%) |
|---|---|---|---|
| Cistus ladaniferus L. Extract*1 | 10 | 100 | 250 |
| Acid mixture*2 | 5 | 100 | 320 |
| Methyl ester mixture*2 | 5 | 100 | 310 |
| Ethyl ester mixture*2 | 5 | 100 | 345 |
| Compound 1 | 5 | 100 | 329 |
| Compound 4 | 5 | 100 | 322 |
| Compound 7 | 5 | 100 | 333 |
| soybean extract*3 | 100 | 100 | 170 |
| Cistus ladaniferus L. Extract*1 | 20 | 0 | 120 |
| Acid mixture *2 | 10 | 0 | 130 |
| Methyl ester mixture*2 | 10 | 0 | 125 |
| Ethyl ester mixture*2 | 10 | 0 | 135 |
| Compound 1*2 | 10 | 0 | 128 |
| compound 4*2 | 10 | 0 | 130 |
| Compound 7*2 | 10 | 0 | 135 |
| soybean extract*3 | 200 | 0 | 108 |
| Panax ginseng extract*4 | 0 | 200 | 110 |

| | | | |
|---|---|---|---|
| *1 prepared in Example 1 *2 prepared in Example 2 *3 prepared in Example 5 *4 manufactured by Maruzen Pharmaceuticals Co., Ltd. | | | |

As is understandable from the results shown in Table 3-1, the plant extract was found to have an activation effect over human neonatal fibroblast NB1RGB, but combined use thereof with the *Panax ginseng* extract, which is a known cell activator, resulted in a more excellent cell activation property. It is therefore expected that the cell activator of the present invention, having *Cistus ladaniferus* L. extract and labdenic acids and methyl esters and ethyl esters thereof as being combined with the *Panax ginseng* extract, applied to the skin exhibits an extremely excellent anti-aging effect, and effectively improves wrinkle and sagging of skin caused by aging, UV exposure and so forth.

### [Example 6: Cream] (out of protection scope)

The individual creams having the compositions shown in Table 4-1 were prepared.

First, ingredients (1) to (6) and (10) were mixed and kept at 70°C, and added with a portion of ingredient (15) again kept at 70°C by heating. The mixture was further added and mixed with ingredients (7) to (9) and (11) to (14), and then cooled to thereby obtain the creams.

Wrinkle improving effect of thus-obtained creams were investigated according to the test method shown below.

**Table 4-1 (Formulations and Results)**

| | Ingredient | Example | | | | | |
|---|---|---|---|---|---|---|---|
| | | 7 | 8 | 9 | 10 | 11 | 12 |
| (1) | bleached bees wax | 6.0 | 6.0 | 6.0 | 6.0 | 6.0 | 6.0 |
| (2) | cetanol | 5.0 | 5.0 | 5.0 | 5.0 | 5.0 | 5.0 |
| (3) | reduced lanolin | 5.0 | 5.0 | 5.0 | 5.0 | 5.0 | 5.0 |
| (4) | squalane | 30.0 | 30.0 | 30.0 | 30.0 | 30.0 | 30.0 |
| (5) | lipophilic glyceryl monostearate | 4.0 | 4.0 | 4.0 | 9.0 | 4.0 | 4.0 |
| (6) | polyoxyethylen (20) sorbitan monolaurate | 2.0 | 2.0 | 2.0 | 2.0 | 2.0 | 2.0 |
| (7) | *Cistus ladaniferus L*. extract*1 | 0.1 | 0.1 | 0.1 | 0.1 | 0.1 | 0.1 |
| (8) | dibutyl hydroxy toluene*2 | - | 0.05 | - | - | - | - |
| (9) | *Sanquisorba officinalis* extract*3 | - | - | 0.5 | - | - | - |
| (10) | 2-ethylhexyl paramethoxycinnamate*4 | - | - | - | 2.5 | - | - |
| (11) | retinol palmitate *5 | - | - | - | - | 0.3 | - |
| (12) | *Tussilago farfara* extract*6 | - | - | - | - | - | 1.0 |
| (13) | antiseptic | q.s. | q.s. | q.s. | q.s. | q.s. | q.s. |
| (14) | perfume | q.s. | q.s. | q.s. | q.s. | q.s. | q.s. |
| (15) | pure water | balance | balance | balance | Balance | balance | balance |
| *8 | effective | 12 | 12 | 14 | 13 | 13 | 12 |
| | little-effective | 3 | 3 | 1 | 1 | 1 | 3 |
| | non-effective | 0 | 0 | 0 | 1 | 1 | 0 |

| | | | | | | | |
|---|---|---|---|---|---|---|---|
| *1 prepared in Example 1 *2 product of Sigma *3 product of Maruzen Pharmaceuticals Co., Ltd. *4 product of BASF *5 product of product of Nippon Roche Ltd. *6 product of Maruzen Pharmaceuticals Co., Ltd. *7 effects of improvement in wrinkle | | | | | | | |

**Table 4-1 (Formulations and Results)**

| | Ingredient | Comparative Example | | | | | | | |
|---|---|---|---|---|---|---|---|---|---|
| | | 9 | 10 | 11 | 12 | 13 | 14 | 15 | 16 |
| (1) | bleached bees wax | 6.0 | 6.0 | 6.0 | 6.0 | 6.0 | 6.0 | 6.0 | 6.0 |
| (2) | cetanol | 5.0 | 5.0 | 5.0 | 5.0 | 5.0 | 5.0 | 5.0 | 5.0 |
| (3) | reduced lanolin | 5.0 | 5.0 | 5.0 | 5.0 | 5.0 | 5.0 | 5.0 | 5.0 |
| (4) | squalane | 30.0 | 30.0 | 30.0 | 30.0 | 30.0 | 30.0 | 30.0 | 30.0 |
| (5) | lipophilic glyceryl monostearate | 4.0 | 4.0 | 4.0 | 4.0 | 4.0 | 4.0 | 4.0 | 4.0 |
| (6) | polyoxyothylen (20) sorbitan monolaurate | 2.0 | 2.0 | 2.0 | 2.0 | 2.0 | 2.0 | 2.0 | 2.0 |
| (7) | *Cistus ladaniferus L.* extract*1 | 0.2 | - | - | - | - | - | - | - |
| (8) | dibutyl hydroxy toluene*2 | - | - | 0.1 | - | - | - | - | - |
| (9) | *Sanguisorba officinalis* extract*3 | - | - | - | 1.0 | - | - | - | - |
| (10) | 2-ethylhexyl paramethoxycinnamate *4 | - | - | - | - | 5.0 | - | - | - |
| (11) | retinol palmitate *5 | - | - | - | - | - | 0.6 | - | - |
| (12) | *Tussilago farfara* extract*6 | - | - | - | - | - | - | 2.0 | - |
| (13) | antiseptic | q.s. | q.s. | q.s. | q.s. | q.s. | q.s. | q.s. | q.s. |
| (14) | perfume | q.s. | q.s. | q.s. | q.s. | q.s. | q.s. | q.e. | q.s. |
| (15) | pure water | balance | balance | balance | Balance | balance | balance | balance | balance |
| *7 | effective | 9 | 5 | 4 | 3 | 2 | 3 | 2 | 0 |
| | little-effective | 5 | 8 | 5 | 5 | 6 | 6 | 6 | 5 |
| | non-effective | 1 | 2 | 6 | 7 | 7 | 6 | 7 | 10 |

| | | | | | | | | | |
|---|---|---|---|---|---|---|---|---|---|
| *1 prepared in Example 1 *2 product of sigma *3 product of Maruzen Pharmaceuticals Co., Ltd. *4 product of BASF *5 product of product of Nippon Roche Ltd. *6 product of Maruzen Pharmaceuticals Co., Ltd. *7 effects of improvement in wrinkle | | | | | | | | | |

### (Test Method)

A panel of 15 female subjects aged from 35 to 59 were employed for each of the sample creams, and made them apply a proper quantity of the creams on their faces after washing twice a day, in the morning and at night, over 12 weeks. Effects of improvement in wrinkle by the application were evaluated based on the criteria below:

| (Criteria for Evaluation) | |
|---|---|
| <Evaluation> | <Description> |
| Effective | wrinkle of skin became non-distinctive |
| Little effective | wrinkle of skin became a little non-distinctive |
| Non-effective | remained unchanged |

It was made clear from the results shown in Table 4-1 that, when applied to the skin, the creams of the present invention in which *Cistus ladaniferus* L. extract and the individual ingredients as ingredient (B) were used together were more successful in improving "wrinkle" or the like of the skin, and making the skin more tense and clear, as compared with the comparative products.

### [Example: 7] Melanin Formation Suppression and Cell Survival Rate Test based on Cell Culture

Murine cultured B16 melanoma cells were used. An appropriate quantity of a 10% FBS-containing MEM medium was placed in two 6-well plates, the B16 melanoma cells were seeded therein and allowed to stand at 37°C under a carbon dioxide concentration of 5 vol%. Next day, a sample preparation solution was added and mixed therewith so as to adjust the final concentrations of the *Cistus ladaniferus* L. extract obtained in Example 1, and labdenic acids and methyl esters and ethyl esters thereof obtained in Example 2 to 0 (reference), 5 and 10 µg/mL, and of *Glycyrrhiza glabra* extract (product of Maruzen Pharmaceuticals Co., Ltd.), which is a known whitening agent, to 0 and 100 µg/mL. The medium was exchanged on the fifth day of culture, and the sample preparation solution was added again. The medium was removed next day, the cells were collected from one plate after washing them using a phosphate buffer (pH7), and degree of whitening of the cultured B16 melanoma cells was evaluated according to the criteria shown below.

Similar test was conducted, as a comparative example, also using *Coix lachryma-jobi* extract (100 µg/mL), already known to have a suppressive effect over melanin formation.

*Coixlachryma-jobi* extract was obtained by adding 100 mL of a 70 vol% water-containing ethanol to 10 g of *Coixlachryma-jobi* (Japan Pharmacopoeia), carrying out extraction at room temperature for 3 days, and by filtering the mixture. Dry solid content of the *Coixlachryma-jobi* extract was found to be 0.8%.

### (Criteria for Judgment)

++: distinctively stronger whiteness over the reference;
+: apparently stronger whiteness over the reference;
±: slightly stronger whiteness over the reference; and
-: remained unchanged.

On the other plate, the cells were fixed with formalin, and dyed by adding an 1% crystal violet solution. Cell survival rates for the individual sample concentrations were measured using a monocellator (product of Olympus Corporation). Results are shown in Table 1-2.

**Table 1-2 (Results)**

| | Final concentration (µ g/mL) | Additional concentration of *Glycyrrhiza glabra* extract (µg/mL) | Degree of whiteness | Cell survival rate (%) |
|---|---|---|---|---|
| Cistus ladaniferus L. Extract*1 | 10 | 100 | ++ | 102 |
| Acid mixture*2 | 5 | 100 | ++ | 108 |
| Methyl ester mixture*2 | 5 | 100 | ++ | 97 |
| Ethyl ester mixture*2 | 5 | 100 | ++ | 93 |
| Compound 1 | 5 | 100 | ++ | 95 |
| Compound 4 | 5 | 100 | ++ | 96 |
| Compound 7 | 5 | 100 | ++ | 98 |
| Coix lachrymal-jobi extract*3 | 100 | 100 | ++ | 97 |
| Cistus ladaniferus L. Extract*1 | 20 | 0 | + | 100 |
| Acid mixture *2 | 10 | 0 | + | 92 |
| Methyl ester mixture*2 | 10 | 0 | + | 90 |
| Ethyl ester mixture*2 | 10 | 0 | + | 90 |
| Compound 1*2 | 10 | 0 | + | 94 |
| Compound 4*2 | 10 | 0 | + | 93 |
| Compound 7*2 | 10 | 0 | + | 98 |
| Coix lachrymal-jobi extract*3 | 200 | 0 | ± | 100 |
| *Glycyrrhiza glabra* extract*4 | 0 | 200 | ± | 100 |

| | | | | |
|---|---|---|---|---|
| *1 prepared in Example 1 *2 prepared in Example 2 *3 prepared in Example 3 *4 manufactured by Maruzen Pharmaceuticals Co., Ltd. | | | | |

### Table 1-2 (Results)

As is understandable from the results shown in Table 1-2, each of *Cistus ladaniferus* L. extract and labdenic acids and methyl esters and ethyl esters thereof were found to have a suppressive effect over melanin formation even when used in a singular manner, and to have only a small toxicity over the cultured B16 melanoma cell. It was also found that combined use with *Glycyrrhiza glabra* extract, which is a known whitening agent, resulted in a synergistic whitening effect, as compared with the case of singular use. It is therefore expected that application of formulations of *Cistus ladaniferus* L. extract and labdenic acids and methyl esters and ethyl esters thereof, combined with *Glycyrrhiza glabra* extract, exhibits an extremely excellent suppressive effect over melanin formation, effectively suppresses sunburn-induced darkening of skin, pigmented spots, freckles and so forth, and raises whitening and skin brightening effects.

### [Example 8] Method of Manufacutring Blackcurrant Fruit Extract

Ten grams of fruit of blackcurrant (*Ribes nigrum* L.) were crushed, added with 100 mL of a 50 vol% water-containing ethanol, extracted at room temperature for 3 days, and filtered to thereby obtain a blackcurrant fruit extract (dry solid content: 2.4%).

### [Example 9] Method of Preparing Inula britannica Extract

Ten grams of flower of *Inula Britannica* L. were added with 100 mL of a 50 vol% water-containing ethanol, extracted at room temperature for 3 days, and filtered to thereby obtain an *Inula britannica* (dry solid content: 0.5%).

### [Example 10] Method of Preparing Cranberry Fruit Extract

Ten grams of fruit of cranberry (*Vaccinium macrocorpon* Aiton or *Vaccinium oxycoccus*) were added with 100 mL of a 50 vol% water-containing 1,3-butylene glycol, extracted at room temperature for 3 days, and filtered to thereby obtain a cranberry fruit extract (dry solid content: 1.5%).

### [Example 11] Method of Preparing Mucuna birdwoodiana Extract

Ten grams of stem of *Mucuna Birdwoodiana* Tutcher were added with 100 mL of a 50 vol% water-containing ethanol, extracted at room temperature for 3 days, and filtered to thereby obtain a *Mucuna birdwoodiana* extract (dry solid content: 1.5%).

### [Example 12] Method of Preparing betula alba Sap

Pre-blooming tree of *Betula platyphylla* Sukatchuv var. japonica Hara was bored, and an effluent was collected, to thereby obtain *betula alba* sap (dry solid content: 0.8%).

### [Example 13] Method of Preparing Alnus firma Extract

Ten grams of fruit of *Alnus firma* Sieb. et Zucc. were added with 100 mL of a 50 vol% water-containing ethanol, extracted at room temperature for 3 days, and filtered to thereby obtain an *Alnus firma* extract (dry solid content: 1.0%).

### [Example 14] Method of Preparing Cactus Extract

Ten grams of dried and pulverized stem of *Opuntia Streptacantha* were added with 100 mL of purified water, and extracted at 70°C for 8 hours. The mixture was cooled and filtered, the filtrate was concentrated to dryness, added with 100 g of a 30 vol% water-containing 1,3-butylene glycol for solubilization, to thereby obtain a cactus extract (dry solid content: 1.0%).

### [Example 15] Method of Preparing Momordica grosvenorii Extract

Ten grams of dried fruit of *Momordica grosvenorii* Swingle were added with 100 mL of a 50 vol% water-containing ethanol, extracted at room temperature for 3 days, and filtered to thereby obtain a *Momordica grosvenorii* extract (dry solid content: 1.3%).

### [Example 16: Cream]

The individual creams having the compositions shown in Table 2-2 were prepared.

First, ingredients (1) to (6) were mixed and kept at 70°C, and added with ingredient (16) again kept at 70°C by heating. The mixture was further added and mixed with ingredients (7) to (15), and then cooled to thereby obtain the creams.

Whitening and skin brightening effects of thus-obtained creams were investigated according to the test method shown below.

### (Test Method)

A panel of 15 female subjects aged from 27 to 54 were employed for each of the sample creams, and made them apply a proper quantity of the creams on their faces after washing twice a day, in the morning and at night, over 12 weeks, and effects of whitening and skin brightening by the application were evaluated based on the criteria below. Results of the evaluation were represented by the number of subjects in the panel relevant to each evaluation.

**(Criteria for Evaluation)**

| <Evaluation> | <Description> |
|---|---|
| Effective | wrinkle of skin became non-distinctive |
| Little effective | wrinkle of skin became a little non-distinctive |
| Non-effective | remained unchanged |

**Table 2-2 (Formulations and Results)**

| | Ingredient | Example | | | | | |
|---|---|---|---|---|---|---|---|
| | | 1 | 2 | 3 | 4 | 5 | 6 |
| (1) | bleached bees wax | 6.0 | 6.0 | 6.0 | 6.0 | 6.0 | 6.0 |
| (2) | cetanol | 5.0 | 5.0 | 5.0 | 5.0 | 5.0 | 5.0 |
| (3) | reduced lanolin | 5.0 | 5.0 | 5.0 | 5.0 | 5.0 | 5.0 |
| (4) | squalane | 30.0 | 30.0 | 30.0 | 30.0 | 30.0 | 30.0 |
| (5) | lipophilic glyceryl monostearate | 4.0 | 4.0 | 4.0 | 4.0 0 | 4.0 | 4.0 |
| (6) | polyoxyethylen (20) sorbitan monolaurate | 2.0 | 2.0 | 2.0 | 2.0 | 2.0 | 2.0 |
| (7) | *Cistus ladaniferus L.* extract*1 | 0.1 | 0.1 | 0.1 | 0.1 | 0.1 | 0.1 |
| (8) | *Glycyrrhiza glabra* extract*2 | 0.1 | - | - | - | - | - |
| (9) | *Coix lachryma-jobi* extract*3 | - | 0.5 | - | - | - | - |
| (10) | blackcurrant fruit extract *4 | - | - | 0.5 | - | - | - |
| (11) | *Inula britannica* extract*5 | - | - | - | 0.5 | - | - |
| (12) | cranberry fruit extract *6 | - | - | - | - | 0.5 | - |
| (13) | *Momordica grosvenorii* extract*7 | - | - | - | - | - | 0.5 |
| (14) | antiseptic | q.s. | q.s. | q.s. | q.s. | q.s. | q.s. |
| (15) | perfume | q.s. | q.s. | q.s. | q.s. | q.s. | q.s. |
| (16) | pure water | balance | balance | balance | Balance | balance | balance |
| *8 | effective | 13 | 13 | 13 | 12 | 12 | 12 |
| | little-effective | 2 | 2 | 2 | 3 | 2 | 3 |
| | non-effective | 0 | 0 | 0 | 0 | 1 | 0 |

| | | | | | | | |
|---|---|---|---|---|---|---|---|
| *1 prepared in Example 1 *2 product of Maruzen Pharmaceuticals Co., Ltd. *3 prepared in Example 22 *4 prepared in Example 23 *5 prepared in Example 24 *6 prepared in Example 25 *7 Prepared in Example 30 *8 Whitening and skin brightening effect | | | | | | | |

**Table 2-2 (Formulations and Results)**

| | Ingredient | Comparative Example | | | | | | | |
|---|---|---|---|---|---|---|---|---|---|
| | | 1 | 2 | 3 | 4 | 5 | 6 | 7 | 8 |
| (1) | bleached bees wax | 6.0 | 6.0 | 6.0 | 6.0 | 6.0 | 6.0 | 6.0 | 6.0 |
| (2) | cetanol | 5.0 | 5.0 | 5.0 | 5.0 | 5.0 | 5.0 | 5.0 | 5.0 |
| (3) | reduced lanolin | 5.0 | 5.0 | 5.0 | 5.0 | 5.0 | 5.0 | 5.0 | 5.0 |
| (4) | squalane | 30.0 | 30.0 | 30.0 | 30.0 | 30.0 | 30.0 | 30.0 | 30.0 |
| (5) | lipophilic glyceryl monostearate | 4.0 | 4.0 | 4.0 | 4.0 | 4.0 | 4.0 | 4.0 | 4.0 |
| (6) | polyoxyethylen (20) sorbitan monolaurate | 2.0 | 2.0 | 2.0 | 2.0 | 2.0 | 2.0 | 2.0 | 2.0 |
| (7) | *Cistus ladaniferus* L*.* extract*1 | 0.2 | - | - | - | - | - | - | - |
| (8) | *Glycyrrhiza glabra* extract*2 | - | 0.2 | - | - | - | - | - | - |
| (9) | *Coix lachryma-jobi* extract*3 | - | - | 1.0 | - | - | - | - | - |
| (10) | blackcurrant fruit extract *4 | - | - | - | 1.0 | - | - | - | - |
| (11) | *Inula britannica* extract*5 | - | - | - | - | 1.0 | - | - | - |
| (12) | cranberry fruit extract *6 | - | - | - | - | - | 1.0 | - | - |
| (13) | *Momordica grosvenorii* extract*7 | - | - | - | - | - | - | 1.0 | - |
| (14) | antiseptic | q.s. | q.s. | q.s. | q.s. | q.s. | q.s. | q.s. | q.s. |
| (15) | perfume | q.s. | q.s. | q.s. | q.s. | q.s. | q.s. | q.s. | q.s. |
| (16) | pure water | balance | balance | balance | Balance | balance | balance | balance | balance |
| *8 | effective | 7 | 5 | 3 | 4 | 5 | 3 | 7 | 0 |
| | little-effective | B | 7 | 7 | B | 7 | 6 | 6 | 5 |
| | non-effective | 0 | 3 | 5 | 3 | 3 | 6 | 2 | 10 |

| | | | | | | | | | |
|---|---|---|---|---|---|---|---|---|---|
| *1 prepared in Example 1 *2 product of Maruzen Pharmaceuticals Co., Ltd. *3 prepared in Example 22 *4 prepared in Example 23 *5 prepared in Example 24 *6 prepared in Example 25 *7 Prepared in Example 30 *8 Whitening and skin brightening effect | | | | | | | | | |

### (Compositions and Results)

It was made clear from the results shown in Table 2-2 that, when applied to the skin, the creams of the present invention in which *Cistus ladaniferus* L. extract and various ingredients as ingredient (B2) (ingredients (8) to (13)) were used together were more successful in preventing and improving "darkening" or the like of the skin, and in making the skin beautiful, as compared with the comparative products.

### [Example 17: Cell Activation Test based on Cell Culture]

Human neonatal fibroblast NB1RGB was used. An appropriate quantity of medium was placed in a 24-well plate, the fibroblast NB1RGB was seeded therein and allowed to stand at 37°C under a carbon dioxide concentration of 5 vol%. Next day, a sample preparation solution was added and mixed therewith so as to adjust the final concentrations of the *Cistus ladaniferus* L. extract obtained in Example 1, and labdenic acids and methyl esters and ethyl esters thereof obtained in Example 2 to 0 (reference), 5 and 10 µg/mL, and of cactus extract, which is a known cell activator, to 0 and 100 µg/mL. The medium was exchanged on the fourth day of culture, and the sample preparation solution was added again. The medium was removed next day, the cells were washed using a phosphate buffer and collected, and evaluated in terms of cell proliferation rate based on comparison of the number of fibroblast NB1RGB cells grown in the individual sample preparation solutions with that of the reference.

Similar test was conducted, as a comparative example, also using soybean extract (100 µg/mL), already known to have a cell activation effect. Soybean extract was obtained by adding 100 mL of a 70 vol% water-containing ethanol to 10 g of soybean seed, carrying out extraction at room temperature for 3 days, and by filtering the mixture. Dry solid content of the soybean extract was found to be 0.5%.

### (Criteria for Evaluation)

The number of cells grown in each sample preparation solution was compared with the number of cells of the reference, and cell activation effect was evaluated using cell proliferation ratio as an index. The number of cells was counted using a blood cell counter plate.

**Table 3-2 (Results)**

| | Final concentration (µg/mL) | Additional concentration of cactus of cactus extract (µg/mL) | Cell-activation rate (%) |
|---|---|---|---|
| Cistus ladaniferus L. Extract*1 | 10 | 100 | 230 |
| Acid mixture*2 | 5 | 100 | 290 |
| Methyl ester mixture*2 | 5 | 100 | 305 |
| Ethyl ester mixture*2 | 5 | 100 | 300 |
| Compound 1 | 5 | 100 | 310 |
| Compound 4 | 5 | 100 | 315 |
| Compound 7 | 5 | 100 | 170 |
| Soybean extract*3 | 100 | 100 | 120 |
| Cistus ladaniferus L. Extract*1 | 20 | 0 | 130 |
| Acid mixture *2 | 10 | 0 | 125 |
| Methyl ester mixture*2 | 10 | 0 | 135 |
| Ethyl ester mixture*2 | 10 | 0 | 135 |
| Compound 1*2 | 10 | 0 | 128 |
| Compound 4*2 | 10 | 0 | 130 |
| Compound 7*2 | 10 | 0 | 135 |
| Soybean extract*3 | 200 | 0 | 108 |
| cactus extract *4 | 0 | 200 | 110 |

| | | | |
|---|---|---|---|
| *1 prepared in Example 1 *2 prepared in Example 2 *3 prepared in Example 5 *4 prepared in Example 29 | | | |

As is understandable from the results shown in Table 3-1, the plant extract was found to have an activation effect over human neonatal fibroblast NB1RGB, but combined use thereof with the cactus extract, which is a known cell activator, resulted in a more excellent cell activation property. It is therefore expected that the cell activator of the present invention, having *Cistus ladaniferus* L. extract and labdenic acids and methyl esters and ethyl esters thereof as being combined with the cactus extract, applied to the skin exhibits an extremely excellent anti-aging effect, and effectively improves wrinkle and sagging of skin caused by aging, UV exposure and so forth.

### [Example 18: Cream]

The individual creams having the compositions shown in Table 4-2 were prepared.

First, ingredients (1) to (6) were mixed and kept at 70°C, and added with a portion of ingredient (16) again kept at 70°C by heating. The mixture was further added and mixed with ingredients (7) to (15), and then cooled to thereby obtain the creams.

### (Test Method)

A panel of 15 female subjects aged from 35 to 59 were employed for each of the sample creams, and made them apply a proper quantity of the creams on their faces after washing twice a day, in the morning and at night, over 12 weeks. Effects of improvement in wrinkle by the application were evaluated based on the criteria below.

**(Criteria for Evaluation)**

| <Evaluation> | <Description> |
|---|---|
| Effective | wrinkle of skin became non-distinctive |
| Little effective | wrinkle of skin became a little non-distinctive |
| Non-effective | remained unchanged |

**Table 4-2 (Formulations and Results)**

| | Ingredient | Example | | | | | |
|---|---|---|---|---|---|---|---|
| | | 7 | 8* | 9* | 10 | 11 | 12* |
| (1) | bleached bees wax | 6.0 | 6.0 | 6.0 | 6.0 | 6.0 | 6.0 |
| (2) | cetanol | 5.0 | 5.0 | 5.0 | 5.0 | 5.0 | 5.0 |
| (3) | reduced lanolin | 5.0 | 5.0 | 5.0 | 5.0 | 5.0 | 5.0 |
| (4) | squalane | 30.0 | 30.0 | 30.0 | 30.0 | 30.0 | 30.0 |
| (5) | lipophilic glyceryl monostearate | 4.0 | 4.0 | 4.0 | 4.0 | 4.0 | 4.0 |
| (6) | polyoxyethylen (20) sorbitan monolaurate | 2.0 | 2.0 | 2.0 | 2.0 | 2.0 | 2.0 |
| (7) | *Cistus ladaniferus L.* extract*1 | 0.1 | 0.1 | 0.1 | 0.1 | 0.1 | 0.1 |
| (8) | *Mucuna birdwoodiana* extract*2 | 0.5 | - | - | - | - | - |
| (9) | *betula alba sep**3 | - | 5.0 | - | - | - | - |
| (10) | Alnus firma extract*4 | - | - | 0.5 | - | - | - |
| (11) | cactus extract*5 | - | - | - | 1.0 | - | - |
| (12) | astaxanthin*6 | - | - | - | - | 0.02 | - |
| (13) | rutin glucoside *7 | - | - | - | - | - | 0.1 |
| (14) | antiseptic | q.s. | q.s. | q.s. | q.s. | q.s. | q.s. |
| (15) | perfume | q.s. | q.s. | q.s. | q.s. | q.s. | q.s. |
| (16) | pure water | balance | balance | balance | Balance | balance | balance |
| *8 | effective | 11 | 12 | 13 | 12 | 13 | 12 |
| | little-effective | 4 | 3 | 2 | 2 | 2 | 3 |
| | non-effective | 0 | 0 | 0 | 1 | 0 | 0 |

| | | | | | | | |
|---|---|---|---|---|---|---|---|
| *1 prepared in Example 1 *2 prepared in Example 26 *3 prepared in Example 27 *4 prepared in Example 28 *5 prepared in Example 29 *6 product of Sigma *7 product of Toyo Sugar Refining Co., Ltd. *8 effects of improvement in wrinkle *comparative | | | | | | | |

**Table 4-2 (Formulations and Results)**

| | Ingredient | Comparative Example | | | | | | | |
|---|---|---|---|---|---|---|---|---|---|
| | | 9 | 10 | 11 | 12 | 13 | 14 | 15 | 16 |
| (1) | bleached bees wax | 6.0 | 6.0 | 6.0 | 6.0 | 6.0 | 6.0 | 6.0 | 6.0 |
| (2) | cetanol | 5.0 | 5.0 | 5.0 | 5.0 | 5.0 | 5.0 | 5.0 | 5.0 |
| (3) | reduced lanolin | 5.0 | 5.0 | 5.0 | 5.0 | 5.0 | 5.0 | 5.0 | 5.0 |
| (4) | squalane | 30.0 | 30.0 | 30.0 | 30.0 | 30.0 | 30.0 | 30.0 | 30.0 |
| (5) | lipophilic glyceryl monostearate | 4.0 | 4.0 | 4.0 | 4.0 | 4.0 | 4.0 | 4.0 | 4.0 |
| (6) | polyoxyethylen (20) sorbitan monolaurate | 2.0 | 2.0 | 2.0 | 2.0 | 2.0 | 2.0 | 2.0 | 2.0 |
| (7) | *Cistus ladaniferus L.* extract*1 | 0.2 | - | - | - | - | - | - | - |
| (8) | *Mucuna birdwoodiana* extract*2 | - | 1.0 | - | - | - | - | - | - |
| (9) | *betula alba sap*3* | - | - | 10.0 | - | - | - | - | - |
| (10) | Alnus firma extract*4 | - | - | - | 1.0 | - | - | - | - |
| (11) | cactus extract*5 | - | - | - | - | 2.0 | - | - | - |
| (12) | astaxanthin*6 | - | - | - | - | - | 0.04 | - | - |
| (13) | rutin glucoside *7 | - | - | - | - | - | - | 0.2 | - |
| (14) | antiseptic | q.s. | q.s. | q.s. | q.s. | q.s. | q.s. | q.s. | q.s. |
| (15) | perfume | q.s. | q.s. | q.s. | q.s. | q.s. | q.s. | q.s. | q.s. |
| (16) | pure water | balance | balance | balance | Balance | balance | balance | balance | balance |
| *8 | effective | 9 | 4 | 5 | 4 | 3 | 7 | 5 | 0 |
| | little-effective | 5 | 9 | 5 | 5 | 7 | 5 | 6 | 5 |
| | non-effective | 1 | 2 | 5 | 6 | 5 | 3 | 4 | 10 |

| | | | | | | | | | |
|---|---|---|---|---|---|---|---|---|---|
| *1 prepared in Example 1 *2 prepared in Example 26 *3 prepared in Example 27 *4 prepared in Example 28 *5 prepared in Example 29 *6 product of Sigma *7 product of Toyo Sugar Refining Co., Ltd. *8 effects of improvement in wrinkle | | | | | | | | | |

It was made clear from the results shown in Table 4 that, when applied to the skin, the creams of the present invention in which *Cistus ladaniferus* L. extract and various ingredients as ingredient (B) were used together were more successful in improving "wrinkle" or the line of the skin, and in making the skin more tense and beautiful as compared with the comparative products.

### [Example 19: Cream]

Ingredients (1) to (10) below were mixed under heating at 70°C. The mixture was added and mixed with ingredients (11) to (13), (22) and (25) preliminarily mixed under heating at 70°C, further added and mixed with ingredients (14) to (21), (23) and (24), cooled to room temperature, to thereby obtain a cream.

| Ingredients | | (%) |
|---|---|---|
| (1) | cetostearyl alcohol | 3.0 |
| (2) | glycerin fatty acid ester | 2.0 |
| (3) | polyoxyethylene (20) sorbitan monooleate | 1.0 |
| (4) | sorbitan monostearate | 1.0 |
| (5) | sodium N-stearoyl-N-methyltaurin | 0.5 |
| (6) | vaselin | 5.0 |
| (7) | dimethyl polysiloxane | 3.0 |
| (8) | glyceryl tri-2-ethyl hexanate | 20.0 |
| (9) | dl-α-tocoferol | 1.0 |
| (10) | titanium oxide | 1.0 |
| (11) | dipropylene glycol | 10.0 |
| (12) | magnesium L-ascorbyl phosphate | 3.0 |
| (13) | sodium citrate | 0.5 |
| (14) | dipotassium glycyrrhiziate | 0.1 |
| (15) | lactic acid (50% aqueous solution) | 0.1 |
| (16) | *Mucuna birdwoodiana* extract *1 | 0.1 |
| (17) | glucosyl rutin *2 | 0.3 |
| (18) | cactus extract *3 | 0.02 |
| (19) | *Momordica grosvenorii* extract *4 | 0.02 |
| (20) | *betula alba* sap *5 | 5.0 |
| (21) | disodium EDTA | 0.03 |
| (22) | antibacterial agent | surfficient quantity |
| (23) | perfume | surfficient quantity |
| (24) | *Cistus ladaniferus* L. extract *6 | 0.5 |
| (25) | purified water | balance |

| | | |
|---|---|---|
| *1 prepared in Example 26 *2 product of Toyo Sugar Refining Co., Ltd. *3 prepared in Example 29 *4 prepared in Example 30 *5 prepared in Example 27 *6 prepared in Example 1 | | |

### [Example 20: Water-in-Oil Type Cream]

Ingredients (1) to (8) below were mixed under heating at 70°C, the mixture was added and mixed with ingredients (9) to (16) and (18) to (21) preliminarily mixed under heating at 50°C, and further mixed with ingredient (17) to thereby obtain a water-in-oil type cream.

| Ingredients | | (%) |
|---|---|---|
| (1) | hydrogen-added soybean phospholipid | 0.05(2) |
| | polyoxyalkylene-modified organopolysiloxane | 2.0 |
| (3) | cholesterol hydroxystearate | 2.0 |
| (4) | cholesterol | 0.2 |
| (5) | squalane | 2.0 |
| (5) | decamethyl cyclopentasiloxane | 7.0 |
| (7) | ethylene glycol diisooctanate | 15.0 |
| (8) | 4-tertbutyl-4'-methoxydibenzoylmethane | 1.0 |
| (9) | magnesium L-ascorbyl phosphate | 3.0 |
| (10) | sodium citrate | 0.5 |
| (11) | disodium EDTA | 0.05 |
| (12) | ethanol | 2.0 |
| (13) | 1,3-butylene glycol | 5.0 |
| (14) | crystalline cellulose | 2.0 |
| (15) | spherical nylon powder | 1.0 |
| (16) | antibacterial agent | surrficient quantity |
| (17) | perfume | sufficient quantity |
| (18) | *Cistus ladaniferus* L. extract *1 | 0.3 |
| (19) | *Alnus firma* extract *2 | 0.1 |
| (20) | *Momordica grosvenorii* extract *3 | 0.1 |
| (21) | purified water | balance |

| | | |
|---|---|---|
| *1 prepared in Example 1 *2 prepared in Example 28 *3 prepared in Example 30 | | |

Both creams obtained in Example 34 and 35 were found to be excellent in long-term stability, and application of which onto the skin showed excellent preventive and improving effects of darkening, pigmented spots and freckles of skin caused typically by sunburn, and wrinkle and sagging of skin caused by aging, and made the skin beautiful and clear.

### [Example 21: Face Cleanser]

Ingredients (1) to (10) below were mixed under heating, and kept at 70°C. The mixture was cooled to room temperature and further added and mixed with ingredients (11) to (14), to thereby obtain a face cleanser.

| Ingredients | | (%) |
|---|---|---|
| (1) | Trietanolamine N-cocoyl-L-glutamate solution | 30.0 |
| (2) | lauryl dimethylamino acetic acid betain | 10.0 |
| (3) | Coconut fatty acid diethanolamide | 3.0 |
| (4) | Potassium cocoate | 5.0 |
| (5) | stearic acid | 2.0 |
| (6) | glycerin | 20.0 |
| (7) | polyethylene glycol 400 | 5.0 |
| (8) | erythritol | 2.0 |
| (9) | propylene glycol | 10.0 |
| (10) | antibacterial agent | surfficient quantity |
| (11) | perfume | sufficient quantity |
| (12) | *Cistus ladaniferus* L. extract *1 | 1.0 |
| (13) | *Coix lachryma-jobi* extract *2 | 1.0 |
| (14) | purified water | balance |

| | | |
|---|---|---|
| *1 prepared in Example 1 *2 prepared in Example 22 | | |

The obtained face cleanser was found to be a face cleanser excellent in long-term stability, and capable of making a clear skin when applied to the skin.

### [Example 22: Gel Cosmetic Formulation]

Ingredients (1) to (5) and (16) below were mixed under heating, and cooled to room temperature. The mixture is added and mixed with ingredients (6) to (10) premixed under heating at 70°C, and further added and mixed with ingredients (11) to (15), to thereby obtain a gel cosmetic formulation.

| Ingredients (%) | | |
|---|---|---|
| (1) | methyl cellulose | 2.0 |
| (2) | xanthane gum | 1.0 |
| (3) | sodium arginate | 0.2 |
| (4) | Acrylate/C10-30 alkyl acrylate crosspolymer | 0.2 |
| (5) | 1% sodium hyaluronate aqueous solution | 2.0 |
| (6) | glycerin | 10.0 |
| (7) | polyethylene glycol 20000 | 1.0 |
| (8) | methyl glucose | 2.0 |
| (9) | hydrogen-added yolk phospholipid | 0.2 |
| (10) | phytosterol | 0.1 |
| (11) | sodium hydroxide | 0.1 |
| (12) | antibacterial agent | surfficeint quantity |
| (13) | perfume | surfficient quantity |
| (14) | *Cistus ladaniferus* L. extract *1 | 0.001 |
| (15) | blackcurrant fruit extract *2 | 0.3 |
| (16) | purified water | balance |

| | | |
|---|---|---|
| *1 prepared in Example 1 *2 prepared in Example 23 | | |

### [Example 23: Oil Gel Cosmetic Formulation]

Ingredients (1) to (9) below were mixed under heating at 70°C, and then cooled to room temperature. The mixture was added and mixed with ingredients (10) and (16), and further added and mixed with ingredients (11) to (15), to thereby obtain an oil gel cosmetic formulation.

| Ingredients | | (%) |
|---|---|---|
| (1) | polyoxyethylene (20) polyoxypropylene (4) cetyl ether | 1.0 |
| (2) | glyceryl polyoxyethylene (20) triisostearate | 0.2 |
| (3) | Acrylate/C10-30 alkyl acrylate crosspolymer | 0.2 |
| (4) | glycerin | 10.0 |
| (5) | dipropylene glycol | 2.0 |
| (6) | 1,3-butylene glycol | 5.0 |
| (7) | polyoxyethylene (10) methyl glucose | 0.2 |
| (8) | glyceryl tri-2-ethylhexanate | 75.0 |
| (9) | squalane | 2.0 |
| (10) | triethanolamine | 0.1 |
| (11) | antibacterial agent | surrficient quantity |
| (12) | perfume | surfficient quantity |
| (13) | *Cistus ladaniferus* L. extract *1 | 0.005 |
| (14) | astaxanthin *2 | 0.05 |
| (15) | rutin glucoside *3 | 0.05 |
| (16) | purified water | balance |
| *1 | prepared in Example 1 | |
| *2 | product of Sigma | |
| *3 | product of Toyo Sugar Refining Co., Ltd. | |

### [Example 24: Lotion]

A mixture having ingredients (1) to (9) and (12), (16) below dissolved therein was added and mixed with ingredients (10), (11), (13) to (15) and (17) to (21) below, to thereby obtain a lotion.

| Ingredients | | (%) |
|---|---|---|
| (1) | macadamia nut oil | 0.01 |
| (2) | borage oil | 0.01 |
| (3) | cetyl octanate | 0.01 |
| (4) | glyceryl tri-2-ethylhexanate | 0.01 |
| (5) | dl-α-tocoferol acetate | 0.02 |
| (6) | sorbitan sesquioleate | 0.1 |
| (7) | polyoxyethylene (20) sorbitan monooleate | 0.1 |
| (8) | polyoxyethylene (8) alkylether phosphate | 0.2 |
| (9) | ethanol | 10.0 |
| (10) | sorbitol(70% aqueous solution) | 5.0 |
| (11) | glycerin | 1.0 |
| (12) | sodium 2-hydroxy-4-inethoxybenzophenone-5-sulfonate | 0.2 |
| (13) | lactic acid (50% aqueous solution) | 0.1 |
| (14) | sodium lactate (50% aqueous solution) | 0.3 |
| (15) | antibacterial agent | surfficient quantity |
| (16) | perfume | surfficient quantity |
| (17) | *Cistus ladaniferus* L. extract *1 | 0.01 |
| (18) | dipotassium glycyrrhiziate | 0.1 |
| (19) | *Alnus firma* extract *2 | 0.05 |
| (20) | *Glycyrrhiza glabra* extract *3 | 0.05 |
| (21) | purified water | balance |

| | | |
|---|---|---|
| *1 prepared in Example 1 *2 prepared in Example 28 *3 product of Maruzen Pharmaceuticals Co., Ltd. | | |

### [Example 25: Lotion]

A mixture having ingredients (1) to (9) below dissolved therein was added and mixed with ingredients (10) to (16), to thereby obatain a lotion.

| Ingredients | | (%) |
|---|---|---|
| (1) | Sucrose γ-linolate | 0.05 |
| (2) | monoisostearic acid polyoxyethylene (50) hydrogenated castor oil | 1.0 |
| (3) | L-ascorbyl isopalmitate | 0.1 |
| (4) | polyoxyethylene (10) alkylether phosphate | 0.1 |
| (5) | octyl methoxycinnamate | 0.05 |
| (6) | glycerin | 3.0 |
| (7) | L-serine | 0.1 |
| (8) | 1,3-butylene glycol | 5.0 |
| (9) | ethanol | 8.0 |
| (10) | sodium citrate | 0.02 |
| (11) | citric acid | 0.05 |
| (12) | antibacterial agent | surfficient quantity |
| (13) | perfume | surfficient quantity |
| (14) | *Cistus ladaniferus* L. extract *1 | 0.05 |
| (15) | *Inula britannica* extract *2 | 0.2 |
| (16) | purified water | balance |

| | | |
|---|---|---|
| *1 prepared in Example 1 *2 prepared in Example 24 | | |

### [Example 26: Turbid Lotion]

A mixture having ingredients (1) to (10) below dissolved therein was added and mixed with ingredients (11) to (19) preliminarily mixed and dissolved, to thereby obtain a turbid lotion.

| Ingredients | | (%) |
|---|---|---|
| (1) | polyoxyethylene (60) hydrogenated castor oil | 0.7 |
| (2) | sodium polyoxyethylene alkylether phosphate | 0.2 |
| (3) | cholesterol | 0.01 |
| (4) | Hydorogenated egg yolk phospholipids | 0.02 |
| (5) | dimethyl polysiloxane | 0.05 |
| (6) | dl-α-tocoferol acetate | 0.5 |
| (7) | 2-ethylhexyl paramethoxycinnamate | 0.2 |
| (8) | stearyl glycyrrhetinate | 0.1 |
| (9) | ethanol | 15.5 |
| (10) | polyethylene glycol 6000 | 0.2 |
| (11) | citric acid | 0.01 |
| (12) | monohydrogen disodium phosphate | 0.2 |
| (13) | astaxanthin *1 | 0.0001 |
| (14) | antibacterial agent | surfficinet quantity |
| (15) | perfume | surfficient quantity |
| (16) | *Cistus ladaniferus* L. extract *2 | 0.002 |
| (17) | rutin glucoside *3 | 0.001 |
| (18) | blackcurrant fruit extract *4 | 0.1 |
| (19) | purified water | balance |

| | | |
|---|---|---|
| *1 product of Sigma *2 prepared in Example 1 *3 product of Toyo Sugar Refining Co., Ltd. *4 prepared in Example 23 | | |

### [Example 27: Milk lotion]

Ingredients (1) to (8) below were mixed under heating at 70°C. The mixture was added and mixed with ingredients (9) to (11) and (17) premixed under heating at 70°C, and then cooled. The mixture was added and mixed with ingredients (12) and (13) to (16), to thereby obtain a milk lotion.

| Ingredients | | (%) |
|---|---|---|
| (1) | Hydorogenated soybean phospholipid | 3.0 |
| (2) | cholesterol | 0.2 |
| (3) | polyoxyethylene (5) cetyl ether | 0.2 |
| (4) | polyoxyethylene (10) hydrogenated castor oil | 1.0 |
| (5) | cetostearyl alcohol | 2.0 |
| (6) | olive squalane | 5.0 |
| (7) | dipropylene glycol | 7.0 |
| (8) | 1,3-butylene glycol | 5.0 |
| (9) | trimethyl glycine | 2.0 |
| (10) | hydroxypropyl methyl cellulose | 0.1 |
| (11) | carboxyvinyl polymer | 0.2 |
| (12) | potassium hydroxide | 0.1 |
| (13) | antibacterial agent | surfficinet quantity |
| (14) | perfume | surfficient quantity |
| (15) | *Cistus ladaniferus L. extract* *1 | 0.1 |
| (16) | cactus extract *2 | 0.5 |
| (17) | purified water | balance |

| | | |
|---|---|---|
| *1 prepared in Example 1 *2 prepared in Example 29 | | |

### [Example 28: Sunscreen Milk lotion]

Ingredients (1) to (11) below were mixed under heating at 70°C so as to disperse them in a slurry form. The mixture was mixed with ingredients (12) to (14) and (16) to (20) preliminarily mixed and dissolved at 50°C, and further added with ingredient (15), to thereby obtain a sunscreen milk lotion.

| Ingredients | | (%) |
|---|---|---|
| (1) | neopentyl glycol dicaprylate | 10.0 |
| (2) | 2-ethylhexyl p-methoxycinnamate | 5.0 |
| (3) | octamethyl cyclotetrasiloxane | 10.0 |
| (4) | decamethyl cyclopentasiloxane | 10.0 |
| (5) | dimethyl polysiloxane | 5.0 |
| (6) | fine titanium oxide powder | 10.0 |
| (7) | fine zinc oxide powder | 5.0 |
| (8) | polyalkylene-modified organopolysiloxane | 5.0 |
| (9) | stearyl glycyrrhetinate | 0.3 |
| (10) | nylon powder | 2.0 |
| (11) | polyethylene powder | 1.0 |
| (12) | glycerin | 5.0 |
| (13) | ethanol | 5.0 |
| (14) | antibacterial agent | surfficient quantity |
| (15) | perfume | surfficient quantity |
| (16) | *Cistus ladaniferus L. extract* *1 | 0.2 |
| (17) | dipotassium glycyrrhiziate | 0.1 |
| (18) | *Mucuna birdwoodiana* extract *2 | 0.1 |
| (19) | cranberry fruit extract *3 | 0.1 |
| (20) | purified water | balance |

| | | |
|---|---|---|
| *1 prepared in Example 1 *2 prepared in Example 26 *3 prepared in Example 25 | | |

### [Example 29: Water-in-Oil Type Sunscreen Cream]

Ingredients (1) to (8) below were mixed under heating at 70°C. The mixture was added and mixed with ingredients (9) to (13) and (15) to (18) preliminarily mixed under heating at 50°C, and further added with ingredient (14), to thereby obtain a water-in-oil type sunscreen cream.

| Ingredients | | (%) |
|---|---|---|
| (1) | polyoxyalkylene-modified organopolysiloxane | 2.0 |
| (2) | octyl palmitate | 15.0 |
| (3) | decamethyl cyclopentasiloxane | 20.0 |
| (4) | glyceryl tribehenate | 1.0 |
| (5) | fine zinc oxide powder | 12.0 |
| (6) | fine titanium oxide powder | 3.0 |
| (7) | 2-ethylhexyl p-methoxycinnamate | 7.0 |
| (8) | 4-tertbutyl-4'-methoxydibenzoylmethane | 1.0 |
| (9) | astaxanthin *1 | 0.05 |
| (10) | dipropylene glycol | 5.0 |
| (11) | ethanol | 5.0 |
| (12) | polyethylene powder | 3.0 |
| (13) | antibacterial agent | surfficient quantity |
| (14) | perfume | surfficeint quantity |
| (15) | *Cistus ladaniferus* L. extract *2 | 0.2 |
| (16) | *betula alba* sap *3 | 0.2 |
| (17) | *Alnus firma* extract *4 | 0.2 |
| (18) | purified water | balance |

| | | |
|---|---|---|
| *1 product of Sigma *2 prepared in Example 1 *3 prepared in Example 27 *4 prepared in Example 28 | | |

### [Example 30: Lotion]

Ingredients (7) to (12) and (19) were mixed under heating at 50°C, and then cooled to room temperature to thereby obtain a mixture. The mixture was added and mixed with a solution preliminarily obtained by mixing and dissolving ingredients (1) to (6) listed below under heating at 50°C, and ingredients (13) to (18), to thereby obtain a viscous lotion.

| Ingredients | | (%) |
|---|---|---|
| (1) | isostearic acid polyoxyethylene (50) hydrogenated castor oil | 0.2 |
| (2) | Hydorogenated soybean phospholipid | 0.5 |
| (3) | glycerin | 7.0 |
| (4) | dl-α-tocoferol | 0.3 |
| (5) | cholesterol | 0.1 |
| (6) | ethanol | 6.0 |
| (7) | sodium 2-hydroxy-4-methoxybenzophenone-5-sulfonate | 0.2 |
| (8) | magnesium L-ascorbyl phosphate | 0.5 |
| (9) | citric acid | 0.01 |
| (10) | sodium citrate | 0.1 |
| (11) | xanthane gum | 0.1 |
| (12) | methyl cellulose | 0.1 |
| (13) | *Cistus ladaniferus* L. extract *1 | 0.1 |
| (14) | *Glycyrrhiza glabra* extract *2 | 0.1 |
| (15) | *Coix lachryma-jobi* extract *3 | 0.1 |
| (16) | cranberry fruit extract *4 | 0.1 |
| (17) | antibacterial agent | surfficinet quantity |
| (18) | perfume | surfficient quantity |
| (19) | purified water | balance |

| | | |
|---|---|---|
| *1 prepared in Example 1 *2 product of Maruzen Pharmaceuticals Co., Ltd. *3 prepared in Example 22 *4 prepared in Example 25 | | |

### [Example 31: Pack Cosmetic Formulation]

Ingredients (1) to (5) and (19) were mixed under heating at 70°C, cooled to room temperature to thereby obtain a mixture, and the mixture was added and mixed with ingredients (6) to (18)to thereby obtain a pack cosmetic formulation.

| Ingredients | | (%) |
|---|---|---|
| (1) | polyvinyl alcohol | 15.0 |
| (2) | glycerin | 10.0 |
| (3) | polyoxyethylene (10) methyl glucose | 3.0 |
| (4) | glyceryl trioctanate | 5.0 |
| (5) | sodium polyoxyethylene alkylether phosphate | 1.0 |
| (6) | ethanol | 20.0 |
| (7) | kaolin | 2.0 |
| (8) | titanium oxide | 2.0 |
| (9) | algae extract | 0.1 |
| (10) | dipotassium glycyrrhiziate | 0.1 |
| (11) | cactus extract *1 | 0.1 |
| (12) | *betula alba* sap *2 | 1.0 |
| (13) | *Inula britannica* extract *3 | 0.1 |
| (14) | lactic acid (50% aqueous solution) | 0.5 |
| (15) | sodium lactate (50% aqueous solution) | 0.5 |
| (16) | antibacterial agent | surfficient quantity |
| (17) | perfume | surffcinet quantity |
| (18) | *Cistus ladaniferus* L. extract *4 | 0.02 |
| (19) | purified water | balance |

| | | |
|---|---|---|
| *1 prepared in Example 29 *2 prepared in Example 27 *3 prepared in Example 24 *4 prepared in Example 1 | | |

### [Example 32: Liquid Foundation]

Ingredients (1) to (7) below were mixed under heating, the mixture was added with ingredients (13) to (18), and then kept at 70°C. The mixture is added to ingredients (8) to (12) preliminarily mixed and kept at 70°C, and uniformly emulsified. After cooled, ingredients (19) to (22) were further added thereto, to thereby obtian a liquid foundation.

| Ingredients | (%) |
|---|---|
| (1) dipentaerythrit fatty acid ester | 2.0 |
| (2) liquid paraffin | 5.0 |
| (3) stearic acid | 2.0 |
| (4) cetanol | 1.0 |
| (5) self-emulsified glyceryl monostearate | 1.0 |
| (6) 2-ethylhexyl p-methoxycinnamate | 8.0 |
| (7) antibacterial agent | surfficient quantity |
| (8) glycerin | 5.0 |
| (9) triethanolamine | 1.0 |
| (10) carboxy methyl cellulose | 0.2 |
| (11) bentonite | 0.5 |
| (12) purified water | balance |
| (13) titanium oxide | 6.0 |
| (14) fine titanium oxide powder | 2.0 |
| (15) fine zinc oxide powder | 4.0 |
| (16) mica | 2.0 |
| (17) talc | 4.0 |
| (18) coloring pigment | surfficient quantity |
| (19) *Cistus ladaniferus* L. extract *1 | 0.01 |
| (20) *Coix lachryma-jobi* extract *2 | 0.5 |
| (21) *Alnus firma* extract *3 | 1.0 |
| (22) perfume | surfficient quantity |

| | |
|---|---|
| *1 prepared in Example 1 *2 prepared in Example 22 *3 prepared in Example 28 | |

All of various cosmetic formulations obtained in Examples 37 to 47 were found to be excellent in long-term stability, and application of which onto the skin showed excellent preventive and improving effects of darkening, pigmented spots and freckles of skin caused typically by sunburn, and wrinkle and sagging of skin caused by aging, and made the skin beautiful and clear.

### Industrial Applicability

As has been described in the above, the present invention can provide an external preparation for skin excellent in skin brightening effect, and in particular in whitening and/or anti-aging effects. These excellent effects are ascribable to a synergistic effect shown by combined use of the compound represented by formula (1) and a particular class of ingredient, and are distinctively excellent skin brightening, whitening and/or anti-aging effects which cannot be obtained by independent use of the compounds represented by formula (1) or of the particular class of ingredients.

## Claims

1. An external preparation for skin, comprising:
(A) one, or two or more compounds represented by formula (1) below: 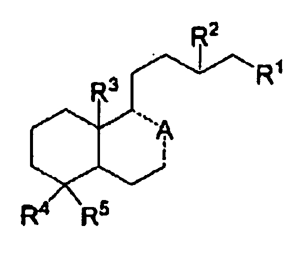 (in formula (1), R¹ represents -CH₂OH or COOR⁶, R⁶ represents hydrogen, a lower alkyl group having the number of carbon atoms of 1 to 3, or a cation capable of forming a salt with COO⁻, each of R² to R⁵ independently represents a hydrogen atom or methyl group, and ....A....represents = C(CH₃) -, -C(CH₃) =, -C (=CH₂) -, -CH(CH₃) - or - C(OH) (CH₃) -); and
(B) one, or two or more medicinal ingredients selected from the group consisting of *Glycyrrhiza glabra* extract, *Coix lachryma-jobi* extract, *blackcurrant fruit* extract, *Inula britannica* extract, *cranberry fruit extract, Mucuna birdwoodiana* extract, *cactus* extract, *Momordica grosvenorii* extract, *and astaxanthin* and its derivates.

2. The external preparation for skin as claimed in claim 1, wherein said compound represented by formula (1) is a compound, or a compound prepared from said compound extracted from one, or two or more plants selected from the plant group consisting of *Cistaceae* including *Cistus ladiniferus L., Cistus creticus* L., *Cistus monoperiensis* L. and *Cistus salvifolius*.

3. The external preparation for skin as claimed in claim 1, being blended with one, or two or more extracts, containing said compound represented by formula (1), selected from the plant group consisting of *Cistaceae* including *Cistus ladaniferus* L., *Cistus creticus* L., *Cistus monoperiensis* L. and *Cistus salvifolius*.

4. Non-therapeutic Use of the external preparation according to any one of claims 1 to 3, for skin whitening.

5. Use of the external preparation for skin anti-aging according to any one of claims 1 to 3 for skin anti-aging.

## Patentansprüche

1. Äußerliches Präparat für die Haut, umfassend:
(A) eine oder zwei oder mehrere Verbindungen, dargestellt anhand der nachstehenden Formel (1): 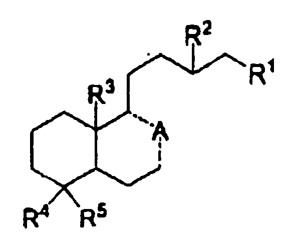 (in Formel (1) steht R¹ für -CH₂OH oder COOR⁶, R⁶ bedeutet Wasserstoff, eine Niederalkyl-Gruppe, bei der die Anzahl der Kohlenstoffatome 1 bis 3 beträgt, oder ein Kation, das mit COO- ein Salz bilden kann, R² bis R⁵ bedeuten unabhängig voneinander jeweils ein Wasserstoffatom oder eine Methyl-Gruppe, und ··· A··· steht für =C(CH₃)-, -C(CH₃)=, -C(=CH₂)-, -CH(CH₃)-oder-C(OH)(CH₃)-; und
(B) einen oder zwei oder mehrere medizinische Bestandteile, ausgewählt aus der Gruppe bestehend aus *Glycyrrhiza* glabra-Extrakt, *Coix lachryma-jobi*-Extrakt, Fruchtextrakt der Schwarzen Johannisbeere, *Inula britannica*-Extrakt, Fruchtextrakt der Moosbeere, *Mucuna birdwoodiana*-Extrakt, Kaktus-Extrakt, *Momordica grosvenorii*-Extrakt und Astaxanthin und dessen Derivaten.

2. Äußerliches Präparat für die Haut nach Anspruch 1, wobei die durch Formel (1) dargestellte Verbindung eine Verbindung oder eine aus dieser Verbindung hergestellte Verbindung ist, die aus einer oder zwei oder mehreren Pflanzen extrahiert wird, die ausgewählt sind aus der Pflanzengruppe bestehend aus *Cistaceae,* darunter *Cistus ladiniferus* L., *Cistus creticus* L., *Cistus monoperiensis* L. und *Cistus salvifolius.*

3. Äußerliches Präparat für die Haut nach Anspruch 1, das mit einem oder zwei oder mehreren Extrakten gemischt wird, welche die durch Formel (1) dargestellte Verbindung enthalten und ausgewählt sind aus der Pflanzengruppe bestehend aus *Cistaceae,* darunter *Cistus ladiniferus L., Cistus creticus* L.*, Cistus monoperiensis* L. und *Cistus salvifolius.*

4. Nichttherapeutische Verwendung des äußerlichen Präparats nach einem der Ansprüche 1 bis 3 zur Aufhellung der Haut.

5. Verwendung des äußerlichen Präparats nach einem der Ansprüche 1 bis 3 gegen die Alterung der Haut.

## Revendications

1. Préparation externe pour la peau, comprenant :
(A) un ou deux composés ou plus représentés par la formule (1) ci-dessous : 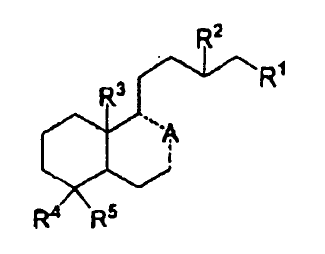 (dans la formule (1), R¹ représente -CH₂OH ou COOR⁶ R⁶ représente un atome d'hydrogène, un groupe alkyle inférieur ayant un nombre d'atomes de carbone de 1 à 3, ou un cation capable de former un sel avec COO⁻, chacun de R² à R⁵ représente indépendamment un atome d'hydrogène ou un groupe méthyle, et ...A... représente =C (CH₃) -, -C(CH₃)=, -C(=CH₂)-, -CH(CH₃)- ou
- C(OH)(CH₃)-) ; et
(B) un ou deux composants médicaux ou plus choisis dans le groupe constitué d'extrait de *Glycyrrhiza glabra*, d'extrait de *Coix lachryma-jobi*, d'extrait de cassis, d'extrait de *Inula britannica*, d'extrait d'airelle, d'extrait de *Mucuna birdwoodiana*, d'extrait de cactus, d'extrait de *Momordica grosvenorii* et d'astaxanthine et de ses dérivés.

2. Préparation externe pour la peau selon la revendication 1, dans laquelle ledit composé représenté par la formule (1) est un composé, ou un composé préparé à partir dudit composé extrait d'une ou de deux plantes ou plus choisies dans le groupe de plantes constitué de Cistacées y compris *Cistus ladaniferus L., Cistus creticus L., Cistus monoperiensis L.* et *Cistus salvifolius*.

3. Préparation externe pour la peau selon la revendication 1, étant mélangée avec un ou deux extraits ou plus, contenant ledit composé représenté par la formule (1), choisis dans le groupe de plantes constitué de Cistacées y compris *Cistus ladaniferus L., Cistus creticus L., Cistus monoperiensis L.* et *Cistus salvifolius.*

4. Utilisation non thérapeutique de la préparation externe selon l'une quelconque des revendications 1 à 3, pour le blanchiment de la peau.

5. Utilisation de la préparation externe pour un antivieillissement de la peau selon l'une quelconque des revendications 1 à 3 pour un antivieillissement de la peau.
